# EUROPEAN PATENT APPLICATION

(11) **EP 4 503 041 A1**
(43) Date of publication of application: **05.02.2025**
(21) Application number: 24191935.6
(22) Date of filing: 31.07.2024
(51) Int. Cl.: G16C 20/20

(54) **SPECTRUM SYNTHESIS FROM INDIVIDUAL SPECTRA**

(30) Priority: 31.07.2023 US 202318362207
(71) Applicant: FEI Company, Hillsboro, OR 97124 (US)
(72) Inventor: OWEN, Michael James, Brisbane, Queensland (AU)
(74) Representative: Stellbrink & Partner Patentanwälte mbB

(57) **Abstract**

Systems, computer program products and/or computer-implemented methods provided herein relate to synthetically generating a synthesized spectrum for a target composition. A system can comprise a memory that stores computer executable components and a processor that executes the computer executable components stored in the memory. The computer executable components can comprise a determination component that determines a known composition having a same component as a target composition, and a generation component that, based on a synthesized spectrum of the known composition and on a first synthesized spectrum of the target composition, generates a second synthesized spectrum of the target composition.

## Description

### BACKGROUND

Scientific instruments for use in material analysis can aid in determining the makeup and properties of an unknown composition. In one or more example, a scientific instrument providing energy dispersive x-ray spectroscopy (EDS) or analyzing a result of an EDS device can aid in the identification of an unknown composition by aiding in providing one or more spectra comparing incident energy of electrons being employed and peak intensity readings.

### BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 illustrates a block diagram of an example scientific instrument for performing operations, in accordance with one or more embodiments described herein.
FIG. 2 illustrates a flow diagram of an example method of performing operations using the scientific instrument of FIG. 1, in accordance with one or more embodiments described herein.
FIG. 3 illustrates a graphical user interface (GUI) that can be used in the performance of one or more of the methods described herein, in accordance with one or more embodiments described herein.
FIG. 4 illustrates a block diagram of an example computing device that can perform one or more of the methods disclosed herein, in accordance with one or more embodiments described herein.
FIG. 5 illustrates a block diagram of an example, non-limiting system that can provide a process to synthetically generate a synthesized spectrum for a target composition, in accordance with one or more embodiments described herein.
FIG. 6 illustrates a block diagram of another example, non-limiting system that can provide a process to synthetically generate a synthesized spectrum for a target composition, in accordance with one or more embodiments described herein.
FIG. 7 illustrates a process of determining a known composition being a nearest neighbor to a target composition, by the material analysis system of FIG. 5, in accordance with one or more embodiments described herein.
FIG. 8 illustrates a flow diagram of a set of processes for synthetic determination of a synthesized spectrum for a composition, by the material analysis system of FIG. 5, in accordance with one or more embodiments described herein.
FIG. 9 illustrates a general process for generation of a resultant synthesized spectrum off a target composition, by the material analysis system of FIG. 5, in accordance with one or more embodiments described herein.
FIG. 10A illustrates a flow diagram of a set of processes for spectrum determinations, by the material analysis system of FIG. 5, in accordance with one or more embodiments described herein.
FIG. 10B illustrates a set of arrays of quantities of photons indexed over a set of energy ranges of spectrums employed by the material analysis system of FIG. 5, in accordance with one or more embodiments described herein.
FIG. 10C illustrates an initial synthesized spectrum of a target composition, from which one array of the set of FIG. 10B is obtained, in accordance with one or more embodiments described herein.
FIG. 10D illustrates a resultant synthesized spectrum of the target composition, from which one array of the set of FIG. 10B is obtained, in accordance with one or more embodiments described herein.
FIG. 11 illustrates a comparison of a visual result as provided by an existing material analysis approach and a visual result as can be provided by one or more embodiments described herein.
FIG. 12 illustrates a flow diagram of one or more processes that can be performed by the material analysis system of FIG. 5, in accordance with one or more embodiments described herein.
FIG. 13 illustrates a continuation of the flow diagram of FIG. 12 of one or more processes that can be performed by the material analysis system of FIG. 5, in accordance with one or more embodiments described herein.
FIG. 14 illustrates a flow diagram of one or more processes that can be performed by the material analysis system of FIG. 5, in accordance with one or more embodiments described herein.
FIG. 15 illustrates a continuation of the flow diagram of FIG. 14 of one or more processes that can be performed by the material analysis system of FIG. 5, in accordance with one or more embodiments described herein.
FIG. 16 illustrates a flow diagram of one or more processes that can be performed by the material analysis system of FIG. 5, in accordance with one or more embodiments described herein.
FIG. 17 illustrates a continuation of the flow diagram of FIG. 16 of one or more processes that can be performed by the material analysis system of FIG. 5, in accordance with one or more embodiments described herein.
FIG. 18 illustrates a block diagram of example scientific instrument system in which one or more of the methods described herein can be performed, in accordance with one or more embodiments described herein.
FIG. 19 illustrates a block diagram of an example operating environment into which embodiments of the subject matter described herein can be incorporated.
FIG. 20 illustrates an example schematic block diagram of a computing environment with which the subject matter described herein can interact and/or be implemented at least in part.

### SUMMARY

The following presents a summary to provide a basic understanding of one or more embodiments described herein. This summary is not intended to identify key or critical elements, and/or to delineate scope of particular embodiments or scope of claims. Its sole purpose is to present concepts in a simplified form as a prelude to the more detailed description that is presented later. In one or more embodiments, systems, computer-implemented methods, apparatuses and/or computer program products described herein can provide a process to employ known compositions and corresponding measured and synthesized spectra thereof to synthesize a spectrum of a target composition.

In accordance with an embodiment, a system can comprise a memory that stores computer executable components and a processor that executes the computer executable components stored in the memory. The computer executable components comprise a determination component that determines a known composition having a same component as a target composition, and a generation component that, based on a synthesized spectrum of the known composition and on a first synthesized spectrum of the target composition, generates a second synthesized spectrum of the target composition.

In accordance with another embodiment, a computer-implemented method can comprise synthesizing, by a system operatively coupled to a processor, a revised spectrum of a target composition, comprising generating, by the system, an intermediary synthesized relationship being a relationship defining a delta of a synthesized spectrum of a known composition and of a first synthesized spectrum of the target composition, aggregating, by the system, the intermediary synthesized relationship and a measured spectrum of the known composition, and generating, by the system, an aggregated synthesized spectrum based on the aggregating and being the revised spectrum of the target composition.

In accordance with still another embodiment, a computer program product facilitating a process for synthesizing a spectrum of a target composition, the program instructions executable by a processor to cause the processor to synthesize, by the processor, a spectrum of a target composition, wherein the synthesizing comprises causing the processor to determine, by the processor, a delta of first quantities of photons of portions of a synthesized spectrum of a known composition and of second quantities of photons of portions of a first synthesized spectrum of the target composition, resulting in an intermediary synthesized relationship, aggregate, by the processor, third quantities of photons of portions of the intermediary synthesized relationship and fourth quantities of photons of portions of a measured spectrum of the known composition, and generate, by the processor, a second synthesized spectrum of the target composition based on a result of the aggregating.

### DETAILED DESCRIPTION

The following detailed description is merely illustrative and is not intended to limit embodiments and/or application or utilization of embodiments. Furthermore, there is no intention to be bound by any expressed or implied information presented in the preceding Summary section, or in the Detailed Description section. One or more embodiments are now described with reference to the drawings, wherein like reference numerals are utilized to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Various operations can be described as multiple discrete actions or operations in turn, in a manner that is most helpful in understanding the subject matter disclosed herein. However, the order of description should not be construed as to imply that these operations are necessarily order dependent. In particular, these operations can be performed in an order different from the order of presentation. Operations described can be performed in a different order from the described embodiment. Various additional operations can be performed, and/or described operations can be omitted in additional embodiments.

Turning now to the subject of material analysis and the one or more embodiments described herein, when identifying an unknown material using measured spectra, such as from energy dispersive X-ray spectroscopy (EDS) of known materials, or from known database spectra, such as obtained by accessing a known database of known materials, discrimination between material phases (e.g., compositions) can be made by use of one or more spectra of a known composition. This known composition can be used as more than mere comparison, and indeed can be employed by the one or more embodiments described herein to generate a synthesized spectrum for an unknown composition.

As used herein, a "synthesized spectrum" or a "synthetically-generated spectrum" refers to a spectrum that is not measured from a sample of established providence but is instead generated by a computing device without having access to a sample of established providence of the unknown material.

As used herein, a sample of "established providence" is a sample for which material composition and other information, such as component weight percentages, is known with high accuracy.

The synthesized spectrum generated by the one or more embodiments described herein can be generated even if a database being used is not exhaustive and has limited known material information. Indeed, material databases are not typically exhaustive, and inherently cannot be exhaustive. For example, many materials can be defined by a range of chemical formula where one cation can change to another cation. For another example, looking only to minerals, there are approximately five thousand known minerals, a majority of which are rare or merely less often encountered, and thus are often missing from available databases.

In addition, the synthesized spectrum generated by the one or more embodiments described herein can be generated even where such synthesized spectrum has two or more (e.g., multiple) significant peaks (e.g., those one or more peaks being larger than other peaks of the spectrum). In one or more embodiments, these significant peaks can be from the same component (e.g., an atomic element, molecular element, phase of an atomic or molecular element, or combination thereof). This is particularly noted when one of the materials in a multi-component composition is copper, iron or zinc, for example and/or such as when the unknown material contains a high proportion of these elements and, as such, the synthesized spectrum being generated can have a recognizable portion of the spectrum resulting from these elements.

Because the significant peaks can be used to define the unknown material, when two or more peaks have similar output values, discrimination by basic comparison alone can be fraught with difficulty. This can be at least in part due to the inherent error of the values being compared and/or such as when using statistical comparison techniques. Rather, such multiple significant peaks are to be differentiated with high accuracy.

To account for one or more inabilities or deficiencies of existing frameworks (e.g., existing material analysis frameworks employing spectrum comparison), one or more embodiments are described herein that can employ one or more known compositions having known material composition similar to an unknown material composition (e.g., a target composition), to accurately identify the target composition, and determine a synthetically-generated spectrum defining the target composition. Even in cases where a measured spectrum of the target composition has multiple significant peaks, the one or more embodiments described herein can accurately identify the target composition and enable the synthetic generation of the spectrum for the target composition.

Furthermore, the resultant synthesized spectra for various target compositions can be employed as future known compositions for use in generating synthesized spectra for other target compositions. That is, a library of synthetically-derived spectra can be built and employed for further material analysis use, thus allowing for a system to self-improve by becoming more accurate for target composition analysis and identification.

As used herein, an "atomic element" is a chemical species that exists as independent atoms.

As used herein, the phrase "based on" should be understood to mean "based at least in part on," unless otherwise specified.

As used herein, the term "component" can refer to an atomic element, molecular element, phase of an atomic or molecular element, or combination thereof.

As used herein, the term "data" can comprise metadata.

As used herein, the terms "entity," "requesting entity," and "user entity" can refer to a machine, device, component, hardware, software, smart device, party, organization, individual and/or human.

As used herein, a "measured spectrum" is one that has been generated based on measurement of a sample of established providence of a composition.

As used herein, a "molecular element" is a molecular substance that comprises two or more atoms of a single element.

As used herein, the term "phase" can refer to an alteration of an atomic element or molecular element.

As used herein, the term "spectrum" can refer to an X-ray spectrum.

Disclosed herein are scientific instrument systems, as well as related methods, computing devices, and computer-readable media. For example, in one or more embodiments, a system can comprise a memory that stores computer executable components and a processor that executes the computer executable components stored in the memory. The computer executable components comprise a determination component that determines a known composition having a same component as a target composition, and a generation component that, based on a synthesized spectrum of the known composition and on a first synthesized spectrum of the target composition, generates a second synthesized spectrum of the target composition.

As noted above, the term "component" can refer to an atomic element, molecular element, phase of an atomic or molecular element, or combination thereof. Thus, a component can refer to a multi-element material, such as NaOH.

The one or more embodiments disclosed herein can achieve improved performance relative to existing approaches. For example, as noted above, even in cases where a measured spectrum of the target composition has multiple significant peaks, the one or more embodiments described herein can accurately identify the target composition and enable the synthetic generation of the spectrum for the target composition. The embodiments disclosed herein thus provide improvements to scientific instrument technology (e.g., improvements in the computer technology supporting such scientific instruments, among other improvements).

Various ones of the embodiments disclosed herein can improve upon existing approaches to achieve the technical advantages of high accuracy identification of material compositions by using known compositions, and particularly the measured and synthetically-generated spectra thereof, as baselines for generating a highly accurate spectrum defining a target composition. Then, by using this synthetically-generated spectrum (i.e., not a measured spectrum), accurate identification of the target composition can be made. As described above, a "synthetically-generated spectrum" refers to a spectrum that is not measured from a sample of established providence but is instead generated by a computing device without having access to a sample of established providence of the unknown material. That is, generation of the synthetically-generated spectrum can be achieved without generating a measured spectrum of the target composition from a sample of established providence, since providence of the target composition itself has not yet been established.

Such technical advantages are not achievable by routine and existing approaches, and all user entities of systems including such embodiments can benefit from these advantages (e.g., by assisting the user entity in the performance of a technical task, such as generation of a synthesized spectrum as part of and/or separate from identification of a target composition, by means of a guided human-machine interaction process).

The technical features of the embodiments disclosed herein (e.g., the ability to generate a spectrum to identify an unknown composition without a sample of established providence and using spectrum data from known compositions) are thus decidedly unconventional in the field of material analysis, as are combinations of the features of the embodiments disclosed herein. As an example, such combinations can comprise the determination of a nearest neighbor known composition and also the use of spectrum data of the known composition to generate the synthesized spectrum of the unknown composition (e.g., target composition).

As discussed further herein, various aspects of the embodiments disclosed herein can improve the functionality of a computer itself; for example, synthetically-generated spectra can be employed for a next known composition for subsequent identifications of subsequent target compositions. The computational and user interface features disclosed herein do not only involve the collection and comparison of information but apply new analytical and technical techniques to change the operation of the computer-analysis of target compositions. That is, a library of synthetically-derived spectra can be built and employed for further material analysis use, thus allowing for the system to self-improve by becoming more accurate for target composition analysis and identification.

The present disclosure thus introduces functionality that neither an existing computing device, nor a human, could perform. Rather, such existing computing devices would instead require material samples of established providence, which is counter to the identification of an unknown target composition (e.g., when rapidly evaluating plural samples, or even hundreds or thousands of samples, of unknown composition, as in the case of a mining operation, it is not practical to have material samples of established providence for each.

Accordingly, the embodiments of the present disclosure can serve any of a number of technical purposes, such as controlling a specific technical system or process; determining from measurements how to control a machine; digital audio, image, or video enhancement or analysis; separation of material sources in a mixed signal; generating data for reliable and/or efficient transmission or storage; providing estimates and confidence intervals for material samples; or providing a faster processing of sensor data. In particular, the present disclosure provides technical solutions to technical problems, including, but not limited to, determination of a nearest neighbor known composition to a target composition, initial generation of baseline synthesized spectra defining the nearest neighbor known composition, generation of a resultant synthesized spectrum defining the target composition, and identification of the target composition based on the resultant synthesized spectrum.

The embodiments disclosed herein thus provide improvements to material analysis technology (e.g., improvements in the computer technology supporting material analysis, among other improvements).

One or more embodiments are now described with reference to the drawings, where like referenced numerals are used to refer to like drawing elements throughout. In the following description, for purposes of explanation, numerous specific details are set forth in order to provide a more thorough understanding of the one or more embodiments. It is evident in various cases, however, that the one or more embodiments can be practiced without these specific details.

Further, it should be appreciated that the embodiments depicted in one or more figures described herein are for illustration only, and as such, the architecture of embodiments is not limited to the systems, devices and/or components depicted therein, nor to any particular order, connection and/or coupling of systems, devices and/or components depicted therein.

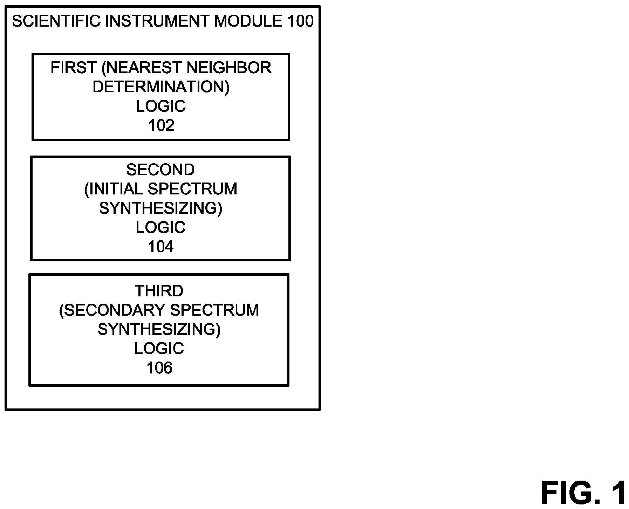

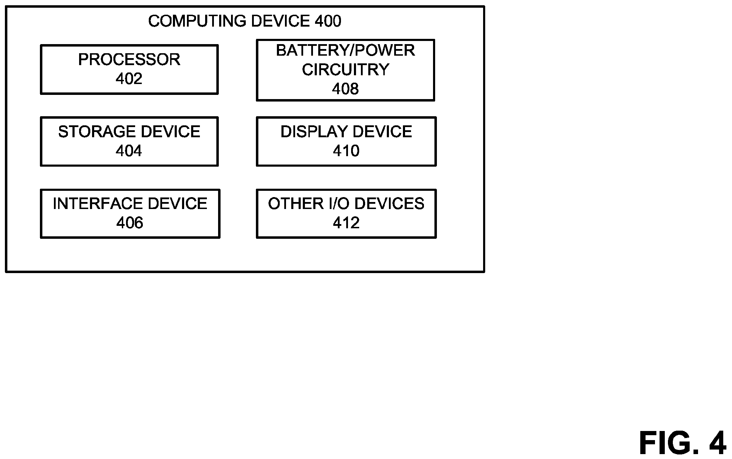

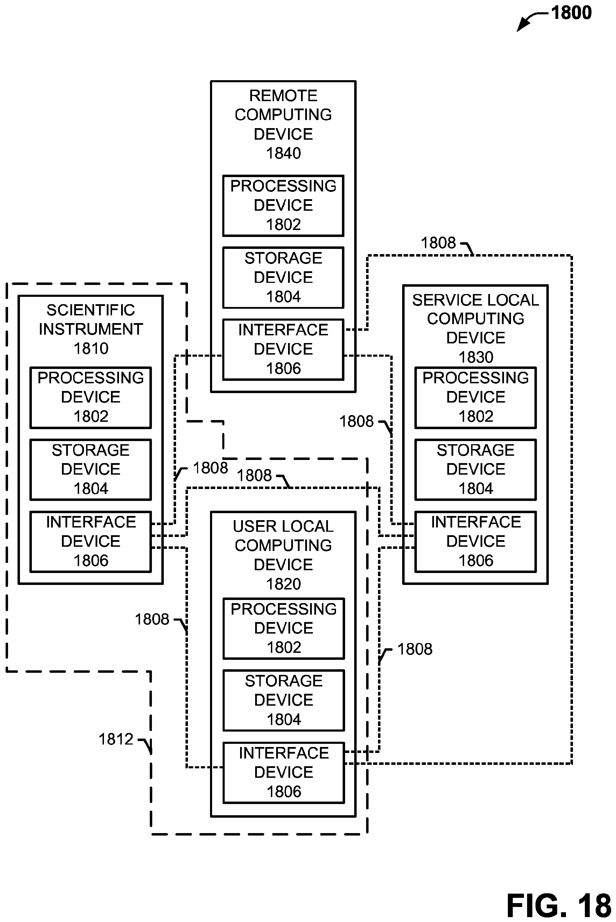

Turning now in particular to the one or more figures, and first to FIG. 1, illustrated is a block diagram of a scientific instrument module 100 for performing material analysis operations using composition spectra resulting synthetic generation and measured from one or more spectroscopy techniques, in accordance with various embodiments described herein. The scientific instrument module 100 can be implemented by circuitry (e.g., including electrical and/or optical components), such as a programmed computing device. The logic of the scientific instrument module 100 can be included in a single computing device or can be distributed across multiple computing devices that are in communication with each other as appropriate. Examples of computing devices that can, singly or in combination, implement the scientific instrument module 100 are discussed herein with reference to the computing device 400 of FIG. 4, and examples of systems of interconnected computing devices, in which the scientific instrument module 100 can be implemented across one or more of the computing devices, is discussed herein with reference to the scientific instrument system 1800 of FIG. 18.

The scientific instrument module 100 can include first logic 102, second logic 104, and third logic 106. As used herein, the term "logic" can include an apparatus that is to perform a set of operations associated with the logic. For example, any of the logic elements included in the module 100 can be implemented by one or more computing devices programmed with instructions to cause one or more processing devices of the computing devices to perform the associated set of operations. In a particular embodiment, a logic element can include one or more non-transitory computer-readable media having instructions thereon that, when executed by one or more processing devices of one or more computing devices, cause the one or more computing devices to perform the associated set of operations. As used herein, the term "module" can refer to a collection of one or more logic elements that, together, perform a function associated with the module. Different ones of the logic elements in a module can take the same form or can take different forms. For example, some logic in a module can be implemented by a programmed general-purpose processing device, while other logic in a module can be implemented by an application-specific integrated circuit (ASIC). In another example, different ones of the logic elements in a module can be associated with different sets of instructions executed by one or more processing devices. A module can omit one or more of the logic elements depicted in the associated drawing; for example, a module can include a subset of the logic elements depicted in the associated drawing when that module is to perform a subset of the operations discussed herein with reference to that module.

The first logic 102 can determine a nearest neighbor of a set of known compositions to a target composition. That is, the first logic 102 can determine a known composition of the set of compositions that has a material composition, such as in terms of atomic element weight percentages (also herein referred to as element weight percentages) or other component weight percentages, that is closest to the target composition, such as also in terms of component weight percentages, of the target composition.

The second logic 104 can generated baseline synthesized spectra for the identified known composition (i.e., the nearest neighbor) and for the target composition, with the term "baseline" referring to the synthesized spectra as being initial spectra for use in further spectrum generation.

The third logic 106 can, using the initial synthesized spectrum of the target composition, the baseline spectrum of the known composition and a measured spectrum of the known composition (e.g., based on a sample of the known composition of known providence), generate a final or resultant synthesized spectrum defining the target composition.

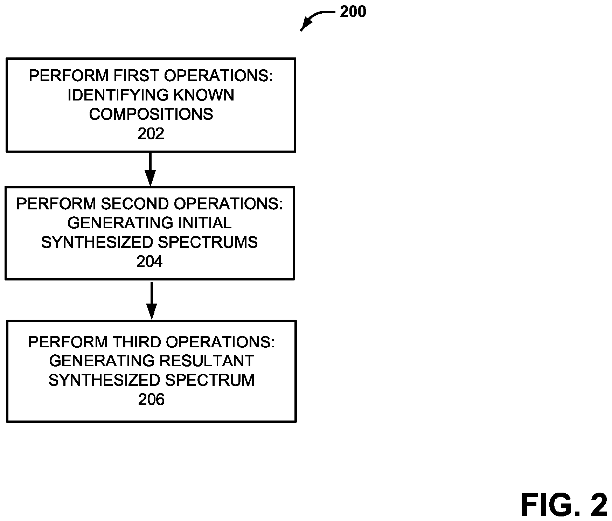

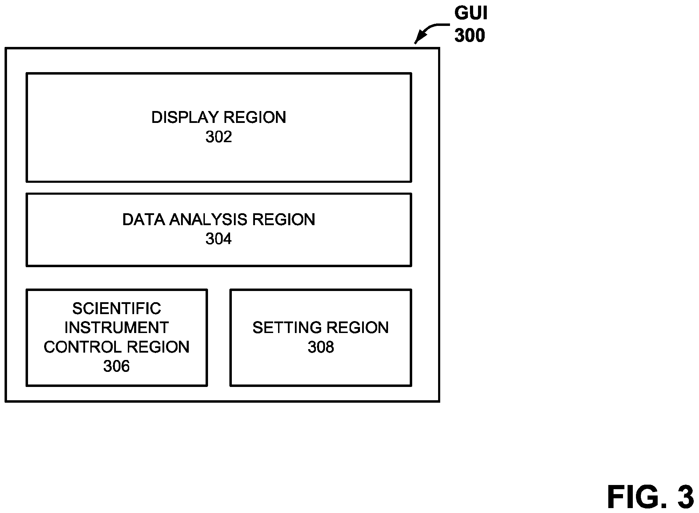

FIG. 2 illustrates a flow diagram of a method 200 of performing operations, by the scientific instrument module 100, in accordance with various embodiments. Although the operations of the method 200 can be illustrated with reference to particular embodiments disclosed herein (e.g., the scientific instrument module 100 discussed herein with reference to FIG. 1, the GUI 300 discussed herein with reference to FIG. 3, the computing device 400 discussed herein with reference to FIG. 4, and/or the scientific instrument system 1800 discussed herein with reference to FIG. 18), the method 200 can be used in any suitable setting to perform any suitable operations. Operations are illustrated once each and in a particular order in FIG. 2, but the operations can be reordered and/or repeated as desired and appropriate (e.g., different operations performed can be performed in parallel, as suitable).

At 202, first operations can be performed. For example, the first logic 102 of the module 100 can perform the first operations 202. The first operations 202 can include identifying the set of known compositions based on the target composition. The first operations 202 further can include determining the nearest neighbor of the set of known compositions to the target composition.

At 204, second operations can be performed. For example, the second logic 104 of the module 100 can perform the second operations 204. The second operations 204 can include generating a baseline synthesized spectrum for the identified known composition (i.e., the nearest neighbor) and generating an initial synthesized spectrum for the target composition. These synthesized spectra can be based on one scaled component spectra of the composition, by running a matrix correction procedure to obtain a compute peak ratio for each component. That is, the synthesized spectra further can be based on a matrix correction procedure of one or more of the sums of component weight percentages of the components of the compositions. The synthesized spectra further can be based on a scaling of the components based on the matrix correction procedure.

As used herein, a matrix correction procedure refers to a procedure, such as a ZAF analysis, inverse ZAF analysis, PAP analysis, Armstrong analysis or XPHI analysis, without being limited thereto, for compensating for one or more deficiencies of traditional quantitative method.

At 206, third operations can be performed. For example, the third logic 106 of the module 100 can perform the third operations 206. The third operations 206 can include generating an intermediary synthesized relationship based on the baseline synthesized spectrum of the known composition and on the initial synthesized spectrum of the target composition, and more particularly based on quantities of photons of portions of the spectra. The third operations 206 also can include generating an aggregated synthesized spectrum based on the intermediary synthesized relationship and on the measured spectrum of the known composition. The third operations 206 further can include generating the resultant synthesized spectrum of the target composition being, or based on, the aggregated synthesized spectrum.

The scientific instrument methods disclosed herein can include interactions with a user entity (e.g., via the user local computing device 1820 discussed herein with reference to FIG. 18). These interactions can include providing information to the user entity (e.g., information regarding the operation of a scientific instrument such as the scientific instrument 1810 of FIG. 18, information regarding a sample being analyzed or other test or measurement performed by a scientific instrument, information retrieved from a local or remote database, or other information) or providing an option for a user entity to input commands (e.g., to control the operation of a scientific instrument such as the scientific instrument 1810 of FIG. 18, or to control the analysis of data generated by a scientific instrument), queries (e.g., to a local or remote database), or other information. In some embodiments, these interactions can be performed through a graphical user interface (GUI) that includes a visual display on a display device (e.g., the display device 410 discussed herein with reference to FIG. 4) that provides outputs to the user entity and/or prompts the user entity to provide inputs (e.g., via one or more input devices, such as a keyboard, mouse, trackpad, or touchscreen, included in the other I/O devices 412 discussed herein with reference to FIG. 4). The scientific instrument system 1800 disclosed herein can include any suitable GUIs for interaction with a user entity.

Turning next to FIG. 3, depicted is an example GUI 300 that can be used in the performance of some or all of the methods described herein, in accordance with various embodiments described herein. As noted above, the GUI 300 can be provided on a display device (e.g., the display device 410 discussed herein with reference to FIG. 4) of a computing device (e.g., the computing device 400 discussed herein with reference to FIG. 4) of a scientific instrument system (e.g., the scientific instrument system 1800 discussed herein with reference to FIG. 18), and a user entity can interact with the GUI 300 using any suitable input device (e.g., any of the input devices included in the other I/O devices 412 discussed herein with reference to FIG. 4) and input technique (e.g., movement of a cursor, motion capture, facial recognition, gesture detection, voice recognition, actuation of buttons, etc.).

The GUI 300 can include a data display region 302, a data analysis region 304, a scientific instrument control region 306, and a settings region 308. The particular number and arrangement of regions depicted in FIG. 3 is merely illustrative, and any number and arrangement of regions, including any desired features thereof, can be included in a GUI 300.

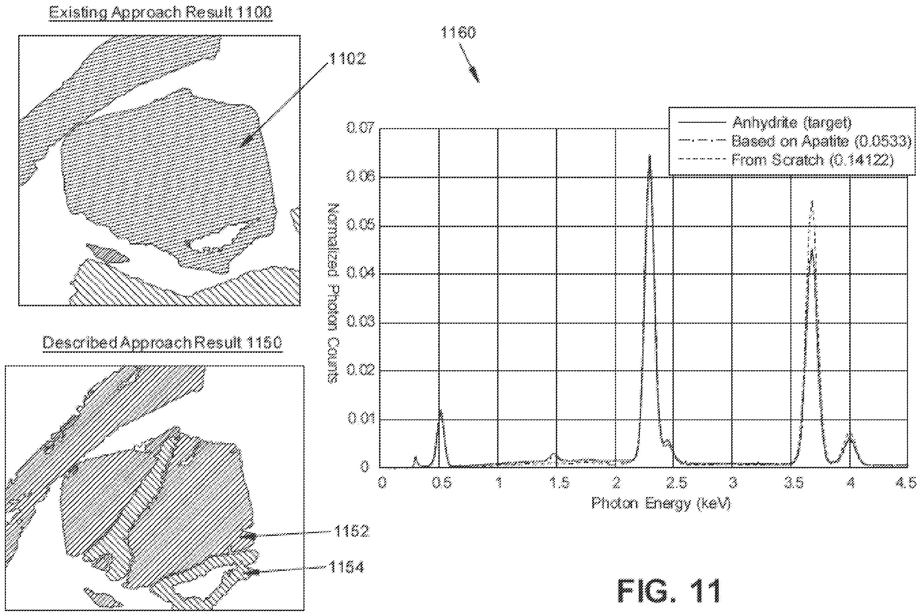

The data display region 302 can display data generated by a scientific instrument (e.g., the scientific instrument 1810 discussed herein with reference to FIG. 18). For example, the data display region 302 can display a resultant synthesized spectrum or data representing such spectrum, such as illustrated at spectrum 1160 of FIG. 11. The spectrum graph 1160 at FIG. 11 has a horizontal x axis depicting the energy of incoming photons in units of kiloelectron volts and is divided into 5 electron volt ranges. The vertical y axis depicts the number of photons acquired within a single range, with the sum of the spectrum being normalized to 1 photon to display measured and synthesized spectra within a same vertical range to highlight the relative heights of element peaks in the range.

The data analysis region 304 can display the results of data analysis (e.g., the results of analyzing the data illustrated in the data display region 302 and/or other data). For example, the data analysis region 304 can display a target composition or identification, such as a visual identification display as illustrated at 1150 of FIG. 11. In one or more embodiments, the data display region 302 and the data analysis region 304 can be combined in the GUI 300 (e.g., to include data output from a scientific instrument, and some analysis of the data, in a common graph or region).

The scientific instrument control region 306 can include options that allow the user entity to control a scientific instrument (e.g., the scientific instrument 1810 discussed herein with reference to FIG. 18). For example, the scientific instrument control region 306 can include one or more controls for selecting and/or verifying selection of a known composition for use in identification of a target composition.

The settings region 308 can include options that allow the user entity to control the features and functions of the GUI 300 (and/or other GUIs) and/or perform common computing operations with respect to the data display region 302 and data analysis region 304 (e.g., saving data on a storage device, such as the storage device 404 discussed herein with reference to FIG. 4, sending data to another user entity, labeling data, etc.). For example, the settings region 308 can include one or more options to alter color, fill or format of illustrations, such as illustrations 1150 and 1160 of FIG. 11.

As noted above, the scientific instrument module 100 can be implemented by one or more computing devices. Accordingly, discussion next turns to FIG. 4, which illustrates a block diagram of a computing device 400 that can perform some or all of the scientific instrument methods disclosed herein, in accordance with various embodiments. In one or more embodiments, the scientific instrument module 100 can be implemented by a single computing device 400 or by multiple computing devices 400. Further, as discussed below, a computing device 400 (or multiple computing devices 400) that implements the scientific instrument module 100 can be part of one or more of the scientific instrument 1810, the user local computing device 1820, the service local computing device 1830, or the remote computing device 1840 of FIG. 18.

The computing device 400 of FIG. 4 is illustrated as having a number of components, but any one or more of these components can be omitted or duplicated, as suitable for the application and setting. As illustrated, these components can include one or more of a processor 402, storage device 404, interface device 406, battery/power circuitry 408, display device 410 and other input/output (I/O) devices 412, as will be described below.

In one or more embodiments, one or more of the components included in the computing device 400 can be attached to one or more motherboards and enclosed in a housing (e.g., including plastic, metal, and/or other materials). In one or more embodiments, some these components can be fabricated onto a single system-on-a-chip (SoC) (e.g., an SoC can include one or more processors 402 and one or more storage devices 404). Additionally, in one or more embodiments, the computing device 400 can omit one or more of the components illustrated in FIG. 4. In one or more embodiments, the computing device 400 can include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface). For example, the computing device 400 can omit a display device 410, but can include display device interface circuitry (e.g., a connector and driver circuitry) to which a display device 410 can be coupled.

The computing device 400 can include the processor 402 (e.g., one or more processing devices). As used herein, the term "processing device" can refer to any device or portion of a device that processes electronic data from registers and/or memory to transform that electronic data into other electronic data that can be stored in registers and/or memory. The processor 402 can include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 400 can include a storage device 404 (e.g., one or more storage devices). The storage device 404 can include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In one or more embodiments, the storage device 404 can include memory that shares a die with a processor 402. In such an embodiment, the memory can be used as cache memory and can include embedded dynamic random access memory (eDRAM) or spin transfer torque magnetic random access memory (STT-MRAM), for example. In one or more embodiments, the storage device 404 can include non-transitory computer readable media having instructions thereon that, when executed by one or more processing devices (e.g., the processor 402), cause the computing device 400 to perform any appropriate ones of or portions of the methods disclosed herein.

The computing device 400 can include an interface device 406 (e.g., one or more interface devices 406). The interface device 406 can include one or more communication chips, connectors, and/or other hardware and software to govern communications between the computing device 400 and other computing devices. For example, the interface device 406 can include circuitry for managing wireless communications for the transfer of data to and from the computing device 400. The term "wireless" and its derivatives can be used to describe circuits, devices, systems, methods, techniques, communications channels, etc., that can communicate data through the use of modulated electromagnetic radiation through a nonsolid medium. The term does not imply that the associated devices do not contain any wires, although in one or more embodiments the associated devices might not contain any wires. Circuitry included in the interface device 406 for managing wireless communications can implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In one or more embodiments, circuitry included in the interface device 406 for managing wireless communications can operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In one or more embodiments, circuitry included in the interface device 406 for managing wireless communications can operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In one or more embodiments, circuitry included in the interface device 406 for managing wireless communications can operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In one or more embodiments, the interface device 406 can include one or more antennas (e.g., one or more antenna arrays) to receipt and/or transmission of wireless communications.

In one or more embodiments, the interface device 406 can include circuitry for managing wired communications, such as electrical, optical, or any other suitable communication protocols. For example, the interface device 406 can include circuitry to support communications in accordance with Ethernet technologies. In one or more embodiments, the interface device 406 can support both wireless and wired communication, and/or can support multiple wired communication protocols and/or multiple wireless communication protocols. For example, a first set of circuitry of the interface device 406 can be dedicated to shorter-range wireless communications such as Wi-Fi or Bluetooth, and a second set of circuitry of the interface device 406 can be dedicated to longer-range wireless communications such as global positioning system (GPS), EDGE, GPRS, CDMA, WiMAX, LTE, EV-DO, or others. In one or more embodiments, a first set of circuitry of the interface device 406 can be dedicated to wireless communications, and a second set of circuitry of the interface device 406 can be dedicated to wired communications.

The computing device 400 can include battery/power circuitry 408. The battery/power circuitry 408 can include one or more energy storage devices (e.g., batteries or capacitors) and/or circuitry for coupling components of the computing device 400 to an energy source separate from the computing device 400 (e.g., AC line power).

The computing device 400 can include a display device 410 (e.g., multiple display devices). The display device 410 can include any visual indicators, such as a heads-up display, a computer monitor, a projector, a touchscreen display, a liquid crystal display (LCD), a light-emitting diode display, or a flat panel display.

The computing device 400 can include other input/output (I/O) devices 412. The other I/O devices 412 can include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 400, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 400 can have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

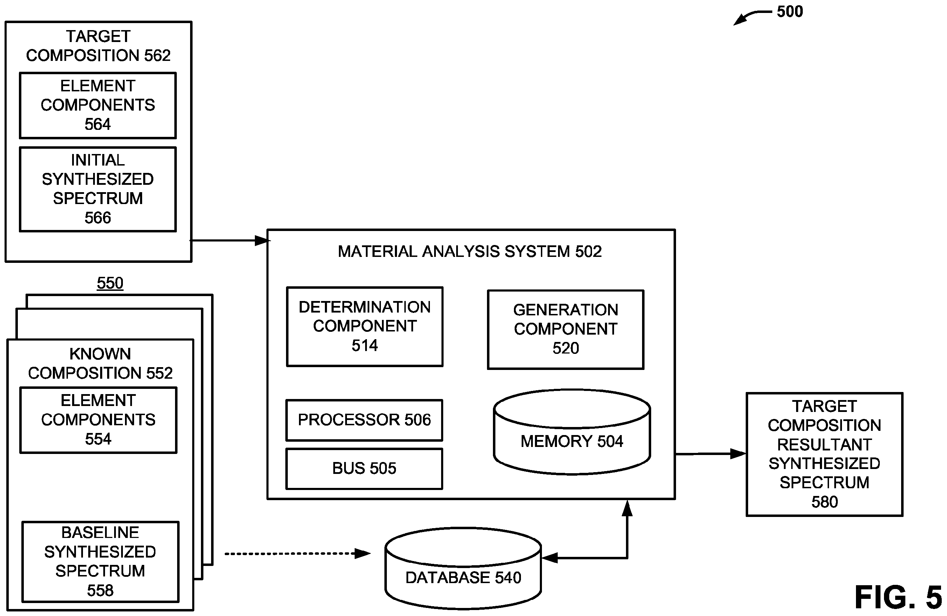

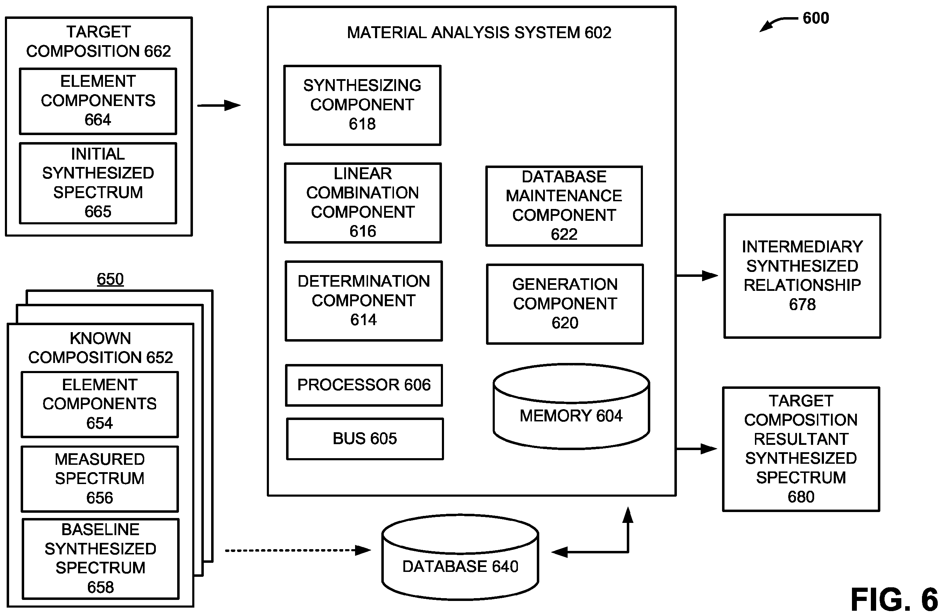

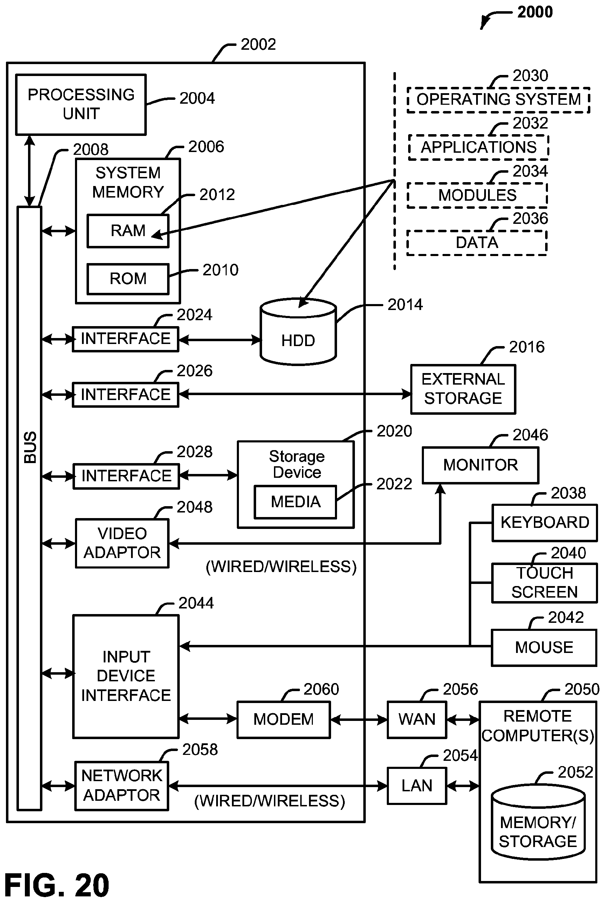

Referring next to FIGS. 5 and 6, in one or more embodiments, the non-limiting systems 500 and/or 600 illustrated at FIGS. 5 and 6, and/or systems thereof, can further comprise one or more computer and/or computing-based elements described herein with reference to a computing environment, such as the computing environment 2000 illustrated at FIG. 20. In one or more described embodiments, computer and/or computing-based elements can be used in connection with implementing one or more of the systems, devices, components and/or computer-implemented operations shown and/or described in connection with FIGS. 5 and/or 6 and/or with other figures described herein.

Turning first to FIG. 5, the figure illustrates a block diagram of an example, non-limiting system 500 that can comprise a material analysis system 502. The material analysis system 502 can facilitate a process to generate a synthesized spectrum of a target composition based on one or more spectra defining a known composition, where the known composition has a material composition related to the target composition. In one or more embodiments, the material analysis system 502 can be at least partially comprised by the computing device 400.

It is noted that the material analysis system 502 is only briefly detailed to provide but a lead-in to a more complex and/or more expansive material analysis system 602 as illustrated at FIG. 6. That is, further detail regarding processes that can be performed by one or more embodiments described herein will be provided below relative to the non-limiting system 600 of FIG. 6.

Still referring to FIG. 5, the material analysis system 502 can comprise at least a memory 504, bus 505, processor 506, determination component 514 and generation component 520. The processor 506 can be the same as the processor 402, comprised by the processor 402 or different therefrom. The memory 504 can be the same as the storage device 404, comprised by the storage device 404 or different therefrom.

Using the above-noted components, the material analysis system 502 can employ both synthetic and measured spectra of a target composition or of a selected known composition to generate a more accurate synthesized spectrum (e.g., a resultant synthesized spectrum) for the target composition than can be generated from initial component composition data for the target composition alone. Indeed, the generation of the resultant synthesized spectrum can be most particularly based on identification of a known composition being a nearest neighbor to the target composition, e.g., having a related material composition, such as using component weight percentages.

The determination component 514 can generally determine a known composition 552 (e.g., a nearest neighbor) having at least one same component 554 as components 564 of a target composition 562. As used herein, components 554 and 564 refer to atomic elements. For example, a known composition 552 and a target composition 562 can each comprise the component silicon (Si).

The generation component 520, based on a baseline synthesized spectrum 558 (e.g., being a baseline for the known composition 552) of the known composition 552 and on an initial (e.g., first) synthesized spectrum 566 of the target composition 562, can generally generate a resultant (e.g., second) synthesized spectrum 580 of the target composition 562. In one or more embodiments, the known composition 552 and data defining the known composition 552 can be obtained from a database 540 communicatively connectable to the material analysis system 502.

The determination component 514 and generation component 520 can be operatively coupled to a processor 506 which can be operatively coupled to a memory 504. The bus 505 can provide for the operative coupling. The processor 506 can facilitate execution of the determination component 514 and generation component 520. The determination component 514 and generation component 520 can be stored at the memory 504.

In general, the non-limiting system 500 can employ any suitable method of communication (e.g., electronic, communicative, internet, infrared, fiber, etc.) to provide communication between the material analysis system 502, a source of data defining the target composition and known composition, and any device associated with a user entity that will use the resultant synthesized spectrum 580 of the target composition 562.

Turning next to FIG. 6, a non-limiting system 600 is illustrated that can comprise a material analysis system 602. Repetitive description of like elements and/or processes employed in respective embodiments is omitted for sake of brevity. Description relative to an embodiment of FIG. 5 can be applicable to an embodiment of FIG. 6. Likewise, description relative to an embodiment of FIG. 6 can be applicable to an embodiment of FIG. 5.

Generally, the material analysis system 602 can facilitate a process to generate a synthesized spectrum of a target composition based on one or more spectra defining a known composition, where the known composition has a material composition related to the target composition. In one or more embodiments, the material analysis system 602 can be at least partially comprised by the computing device 400.

One or more communications between one or more components of the non-limiting system 600 can be provided by wired and/or wireless means including, but not limited to, employing a cellular network, a wide area network (WAN) (e.g., the Internet), and/or a local area network (LAN). Suitable wired or wireless technologies for supporting the communications can include, without being limited to, wireless fidelity (Wi-Fi), global system for mobile communications (GSM), universal mobile telecommunications system (UMTS), worldwide interoperability for microwave access (WiMAX), enhanced general packet radio service (enhanced GPRS), third generation partnership project (3GPP) long term evolution (LTE), third generation partnership project 2 (3GPP2) ultra-mobile broadband (UMB), high speed packet access (HSPA), Zigbee and other 802.XX wireless technologies and/or legacy telecommunication technologies, BLUETOOTH^{®}, Session Initiation Protocol (SIP), ZIGBEE^{®}, RF4CE protocol, WirelessHART protocol, 6LoWPAN (Ipv6 over Low power Wireless Area Networks), Z-Wave, an advanced and/or adaptive network technology (ANT), an ultra-wideband (UWB) standard protocol and/or other proprietary and/or non-proprietary communication protocols.

The material analysis system 602 can be associated with, such as accessible via, a cloud computing environment.

The material analysis system 602 can comprise a plurality of components. The components can comprise a memory 604, processor 606, bus 605, determination component 614, linear combination component 616, synthesizing component 618, generation component 620 and database maintenance component 622. Using these components, the material analysis system 602 can output one or more resultant synthesized spectra defining a target composition 662, or identification of a target composition 662, in response to a query, regarding an unknown material (e.g., the target composition). The query can be requested by an entity, such as an administrator entity of a mining system or other system.

Discussion next turns to the processor 606, memory 604 and bus 605 of the material analysis system 602. For example, in one or more embodiments, the material analysis system 602 can comprise the processor 606 (e.g., computer processing unit, microprocessor, classical processor, quantum processor and/or like processor). In one or more embodiments, a component associated with material analysis system 602, as described herein with or without reference to the one or more figures of the one or more embodiments, can comprise one or more computer and/or machine readable, writable and/or executable components and/or instructions that can be executed by processor 606 to provide performance of one or more processes defined by such component and/or instruction. In one or more embodiments, the processor 606 can comprise the determination component 614, linear combination component 616, synthesizing component 618, generation component 620 and database maintenance component 622.

In one or more embodiments, the material analysis system 602 can comprise the computer-readable memory 604 that can be operably connected to the processor 606. The memory 604 can store computer-executable instructions that, upon execution by the processor 606, can cause the processor 606 and/or one or more other components of the material analysis system 602 (e.g., determination component 614, linear combination component 616, synthesizing component 618, generation component 620 and database maintenance component 622) to perform one or more actions. In one or more embodiments, the memory 604 can store computer-executable components (e.g., determination component 614, linear combination component 616, synthesizing component 618, generation component 620 and database maintenance component 622).

The material analysis system 602 and/or a component thereof as described herein, can be communicatively, electrically, operatively, optically and/or otherwise coupled to one another via a bus 605. Bus 605 can comprise one or more of a memory bus, memory controller, peripheral bus, external bus, local bus, quantum bus and/or another type of bus that can employ one or more bus architectures. One or more of these examples of bus 605 can be employed.

In one or more embodiments, the material analysis system 602 can be coupled (e.g., communicatively, electrically, operatively, optically and/or like function) to one or more external systems (e.g., a non-illustrated electrical output production system, one or more output targets and/or an output target controller), sources and/or devices (e.g., classical and/or quantum computing devices, communication devices and/or like devices), such as via a network. In one or more embodiments, one or more of the components of the material analysis system 602 and/or of the non-limiting system 600 can reside in the cloud, and/or can reside locally in a local computing environment (e.g., at a specified location).

In addition to the processor 606 and/or memory 604 described above, the material analysis system 602 can comprise one or more computer and/or machine readable, writable and/or executable components and/or instructions that, when executed by processor 606, can provide performance of one or more operations defined by such component and/or instruction.

Turning now to the additional components of the material analysis system 602 (e.g., determination component 614, linear combination component 616, synthesizing component 618, generation component 620 and database maintenance component 622), generally, the material analysis system 602 can perform a set of processes that can be separated into three steps: nearest neighbor determination, initial synthesized spectrum generation, and resultant synthesized spectrum generation.

Turning first to the determination component 614, this component can generally determine a known composition 652 (e.g., a nearest neighbor) having one or more same components 654 (e.g., components 654) as components 664 (e.g., components 664) of the target composition 662.

More particularly, based on a set of known compositions 650, such as obtained from a database 640 or one or more other databases, the determination component 614 can determine one composition of the set 650 as being a nearest neighbor of the set 650. The "nearest neighbor" can be a known composition having a composition/makeup most similar to the target composition 662. In one or more embodiments, this determination can be based on component weight percentages. That is, a selected nearest neighbor known composition 652 can have component weight percentages closer to component weight percentages of the target composition than the other known compositions of the set 650 of known compositions. In one or more embodiments, the known composition 652 and data defining the known composition 652 can be obtained from a database 640 communicatively connectable to the material analysis system 602

The nearest neighbor determination by the determination component 614 can generally verify that a delta, of component weight percentages of the known composition 652 and of component weight percentages of the target composition 662, satisfy a selected threshold defining a selected compositional relationship between the known composition 652 and the target composition 662. The selected threshold can be defined by a user entity or by the material analysis system 602.

In one or more embodiments, the selected threshold can generally be defined as being based on an error metric between a pair of vectors comprising component weight percentages.

For example, in one or more embodiments, the selected threshold can be defined as a delta of an output of a sum of absolute deviations of the components of the target composition and of the components of the known composition. In one or more embodiments, the selected threshold can be defined as a delta of an output of a sum of relative deviations of the components of the target composition and of the components of the known composition. An example relative error method can be defined by Error = abs(element₁ - element₂)/element₁, for a single element, for example.

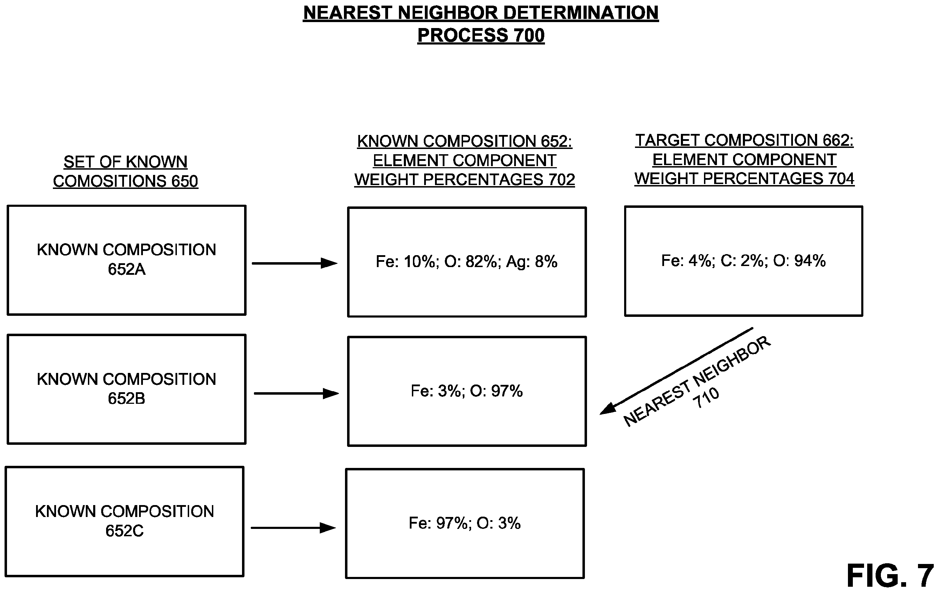

As another example, turning briefly to FIG. 7 and the nearest neighbor determination process 700, this determination by the determination component 614 can be illustrated based on exemplary known compositions 652A, 652B and 652C.

The target composition 662 is illustrated as having component weight percentages 704 of 4% iron (Fe), 2% carbon (C) and 94% oxygen (O). The known composition 652A is illustrated as having component weight percentages 702 of 10% Fe, 82% O and 8% Ag. A delta of these component weight percentages 702 and 704 can be determined as shown generally at Equation 1 and particularly at Equation 2, below, using absolute value deviations. | %FeT - %FeK | and | %QT - %QK | = Δ%Fe and Δ%O; Sum of Deviations = Δ%Fe + Δ%O | 4% - 10% | for Fe, | 94% - 82% | for O, | 2% - 0% | for C and | 8% - 0% | for Ag = 6% Fe, 12% O, 2% C and 8% Ag; Sum of Deviations = 6% + 12% + 2% + 8% = 28%.

In one or more embodiments, elements C and Ag can be omitted.

Differently, the known composition 652B is illustrated as having component weight percentages 702 of 3% Fe and 97% O as compared to the component weight percentages 704 of the target composition 662 of 4% Fe and 2% C and 94% O. Accordingly, a delta of these component weight percentages 702 and 704 can be determined as particularly shown at Equation 3, below, using absolute value deviations.

In one or more embodiments, element C can be omitted. | 4% - 3% | for Fe, | 94% - 97% | for O and | 2% - 0% | for C = 1% Fe, 3% O and 2% C; Sum of Deviations = 1% + 3% + 2% + 2% = 6%.

Accordingly, the separate delta component weight percentages (e.g., separately for O and separately for Fe) for the known composition 652B each are lower than the separate delta component weight percentages for the known composition 652A. Accordingly, the known composition 652/nearest neighbor 710 is the known composition 652B.

Additionally, the sum of deviations (6%) for the known composition 652B is lower than the sum of deviations (28%) for the known composition 652A.

It is noted that known composition 652C has a sum of deviations of 184%.

In one or more other embodiments, another suitable method can be employed that is at least partially based on component weight percentages or that is not based at all on component weight percentages.

Turning next briefly to the linear combination component 616, in a case where no sum of deviations satisfies a selected threshold for the sum of deviations, and/or where the entity desires, an exemplary known composition can be based on a combination (e.g., aggregation) of two or more known compositions. These two or more known compositions can be obtained from same or different databases 640 by the determination component 614. Based on the obtaining of the two or more known compositions, the linear combination component 616 can perform an aggregation, such as of the component weight percentages of the two or more known compositions. In one or more embodiments, the aggregation can comprise averaging first component weight percentages of a first element of the two or more known compositions, averaging second component weight percentages of a second element of the two or more known compositions, and so on. This can result in a combination known composition having separately averaged component weight percentages for each different element of the combination known composition.

For example, when synthesizing the spectrum for the mineral albite (NaAlSi3O8) once can acquire O=48.8%, Na=8.7%, AI=10.3% and Si=32.1%. This can be constructed from a set of three reference compositions (here being minerals) SiO₂, Na₂O and Al₂O₃, for example, with a ratio of approximately 68.7% SiO₂, 19.5% Al₂O₃ and 11.9% Na₂O. These numbers can scale the measured spectra of these references and hence yield a synthetic spectrum that is close to the target. Given that Na₂O is a rare compound, this composition may not be present in an accessible database of known compositions, and the remaining contributions for Na and O might be derived synthetically. The other compounds SiO₂ and Al₂O₃ are common, and thus are more likely to be present in an accessible database.

In such case, the combination known composition can be employed as one of the comparison samples for determining a nearest neighbor 710, as discussed above.

Upon determination of the nearest neighbor 710, a first stage of identification is complete, with the first stage being nearest neighbor determination. A second stage comprises initial synthesized spectrum generation. For this second stage, discussion now turns to the synthesizing component 618.

The synthesizing component 618 generally can synthetically generate a baseline synthesized spectrum 658 for the known composition 652 and an initial synthesized spectrum 665 for the target composition 662. These synthetically generates spectra can be subsequently employed to aid in identifying the target composition 662. That is, the initial synthesized spectrum 665 of the target composition 662 will be an initial estimate of identification of the target composition 662, with subsequent additional processes being performed by the generation component 620 to provide a more accurate identification for the target composition 662.

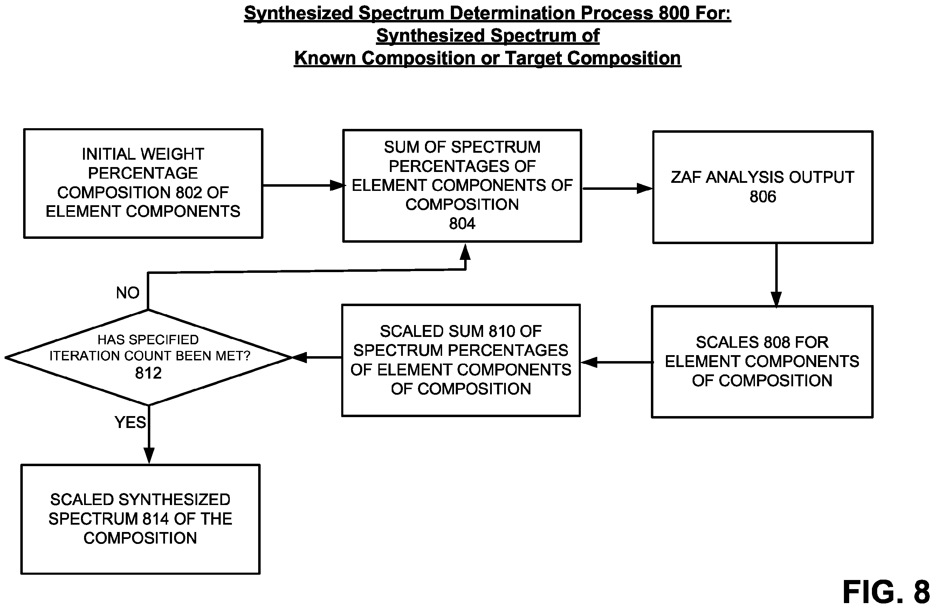

Generally, the baseline synthesized spectrum 658 can be based on sums of component weight percentages of the components of the known composition 652 and on a matrix correction procedure, such as a ZAF analysis, PAP analysis, Armstrong analysis or XPHI analysis, without being limited thereto, to allow for scaling of the sums over a set of iterations of summing and subsequent scaling. That is, turning to FIG. 8 and the synthesized spectrum determination process 800, a first step can comprise determination of initial weight percentage composition 802 of components of the known composition 652. For example, given a known composition having component weight percentages of 3% Fe and 97% O, the 3% Fe spectrum and the 97% O spectrum can be aggregated. The result is a sum of component weight percentages of components of the composition 804.

In one embodiment, a ZAF analysis can be performed on the result 804, with an analysis output 806 resulting therefrom. A ZAF analysis can refer to a quantitative analysis that takes into account three effects of the characteristic X-ray intensity including atomic number (Z) effect, absorption (A) effect, and fluorescence excitation (F) effect.

In one or more other embodiments, a PAP analysis can be employed, as indicated above. The PAP (Pouchou and Pichoir) matrix correction procedure can be used in Energy-Dispersive X-ray Spectroscopy (EDS) to improve the accuracy of quantitative analysis in thin specimens. It addresses X-ray absorption and fluorescence effects that can influence the measured X-ray intensities. The PAP method can involve iteratively calculating correction factors for each element in the sample based on theoretical models and experimental data, enabling researchers to obtain more reliable elemental composition information from EDS spectra of thin materials. The corrections provided by the PAP method help compensate for these effects and lead to more accurate and precise quantitative results in EDS analysis.

The PAP (Pouchou and Pichoir) matrix correction procedure and ZAF (Z (Atomic Number), A (Absorption), F (Fluorescence)) methods can both be used in Energy-Dispersive X-ray Spectroscopy (EDS) for quantitative analysis. A difference between these methods lies in their approach to correcting for X-ray absorption and fluorescence effects. While ZAF methods address these corrections individually, the PAP method combines both the absorption and fluorescence corrections into a single, unified correction scheme. This integration allows the PAP method to provide more accurate and reliable elemental quantification, especially for thin specimens.

As an example, using ZAF analysis (e.g., a ZAF method), the analysis output 806 can provide 1% Fe and 99% O. From this output 806, a set of scales 808 for components of the composition can be generated. In the example below, the set of scales 808 is Fe 3x and O 0.9x. For example, the following steps of Equations 4 and 5 can be performed, with Equation 4 being a general set of steps and Equation 5 continuing the example composition of FE and O. It is noted that the notation below of ZAF analysis can be replaced with one or more matrix correction procedures generally or with another type of matrix correction procedure more particularly.

Equation 4 (performed by synthesizing component 618):
a) Given ZAF analysis output of component weight percentages of elements;
b) Given initial percentages determined for elements;
c) Compute new scale for element 1: initial component weight percentage/ZAF analysis percentage = scale of x (e.g., 0.1X, 2.4X, etc.);
d) Compute new scale for element 2: initial component weight percentage/ZAF analysis percentage = scale of x;
e) Compute new scales for additional elements;
f) Apply new scales to the original percentages: (element 1: initial component weight percentage * scale of x and element 2: initial component weight percentage * scale of x; and
g) Output scaled sum = scaled element 1 and scaled element 2
h) Repeat steps a to f depending on threshold iteration count.

Equation 5 (performed by synthesizing component 618):
a) Given ZAF analysis output 1% Fe and 99% O;
b) Given initial percentages 3% Fe and 97% O;
c) Compute new Fe scale: 3%/1% = 3x;
d) Compute new O scale: 97%/99% = 0.9x;
e) Apply new scales to the original percentages: (Fe: 3%*3x and O: 97%*0.9x); and
f) Output scaled sum = 9% Fe and 95% O.
g) Repeat steps a to f depending on threshold iteration count.

As described above, the scales sum 810 of the component weight percentages of components of the known composition is 9% Fe and 95% O, which is different from the given initial percentages of 3% Fe and 97% O.

In another example, a PAP analysis can be performed to obtain the analysis output 806, scales 808 and scales sum 810.

As described above, the computation of new scales 808 and a resultant scaled sum 810 can be repeated a number of times until satisfying at least a selected threshold iteration count. The threshold can be determined by a user entity or by the synthesizing component 618. For example, as illustrated at decision step 812 of FIG. 8, if the specified iteration count has been met, the synthesized spectrum determination process 800 can proceed to generate and output a scaled synthesized spectrum 814 for the known composition 652 and based on the scaled sum 810 of component weight percentages. If the specified iteration count has not been met, the synthesized spectrum determination process 800 can proceed to re-performing steps outputting the analysis output 806, the scales 808 and scaled sum 810.

It is noted that the output of the synthesized spectrum determination process 800 is the baseline synthesized spectrum 658, which is the final scaled synthesized spectrum 814, after all completed iterations of the process 800.

The same synthesized spectrum determination process 800 can be completed for the target composition 662 to output the initial synthesized spectrum 665, which will be the final scaled synthesized spectrum 814, after all completed iterations of the process 800.

Upon determination of the initial synthesized spectrum 665 of the target composition 662 and of the baseline synthesized spectrum 658 of the known composition 652, a third stage of identification can be performed. The third stage can be generation of a resultant synthesized spectrum 680 (e.g., a revised synthesized spectrum for the target composition 662).

That is, turning next to the generation component 620, this component can generally, based on a baseline synthesized spectrum 658 (e.g., being a baseline for the known composition 652) of the known composition 652 and on an initial (e.g., first) synthesized spectrum 665 of the target composition 662, can generally generate a resultant (e.g., second) synthesized spectrum 680 of the target composition 662. In one or more embodiments, the known composition 652 and data defining the known composition 652 can be obtained from a database 640 communicatively connectable to the material analysis system 602.

However, first, prior to outputting the resultant synthesized spectrum 680 of the target composition 662, two intermediate steps are first performed by the generation component 620.

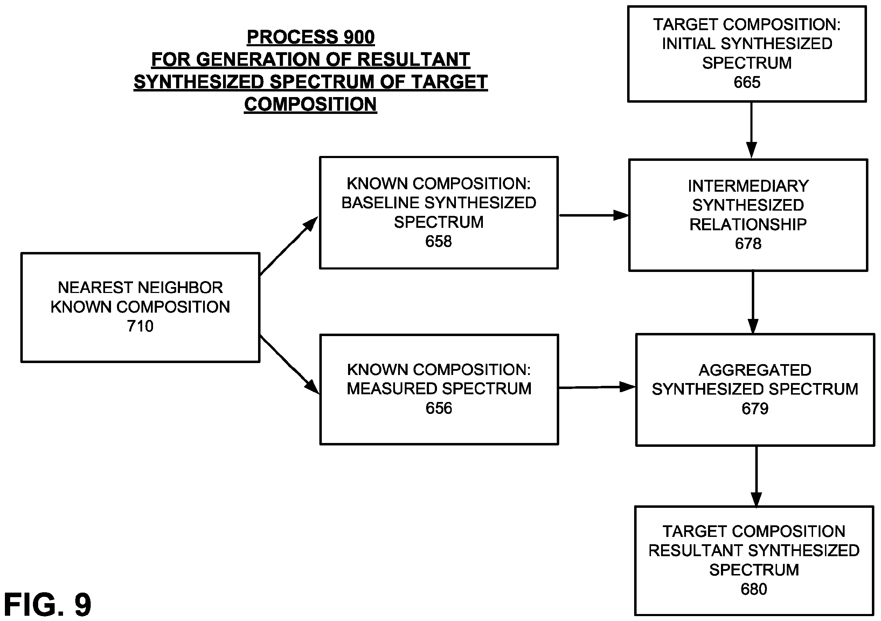

Turning now to FIG. 9 and to a process 900 for generation of the resultant synthesized spectrum of the target composition, the first step comprises generation of the intermediary synthesized relationship 678 by the generation component 620.

That is, based on the nearest neighbor known composition 710, the baseline synthesized spectrum 658 was generated by the synthesizing component 618 for the known composition 652. Further a measured spectrum 656 for the known composition 652 can be obtained, such as by the generation component 620, such as from a database (e.g., database 640). It is noted that the measured spectrum 656 is a spectrum measured by a scientific instrument, such as an EDS device, based on a sample of known providence of the known composition 652 (e.g., a sample for which composition is accurately known).

For generating the intermediary synthesized relationship 678, the initial synthesized spectrum 665 of the target composition 662 and the baseline synthesized spectrum 658 of the known composition 652 can be employed. That is, the intermediary synthesized relationship 678 is determined as being a relationship defined by a delta these two synthesized spectra 665 and 658.

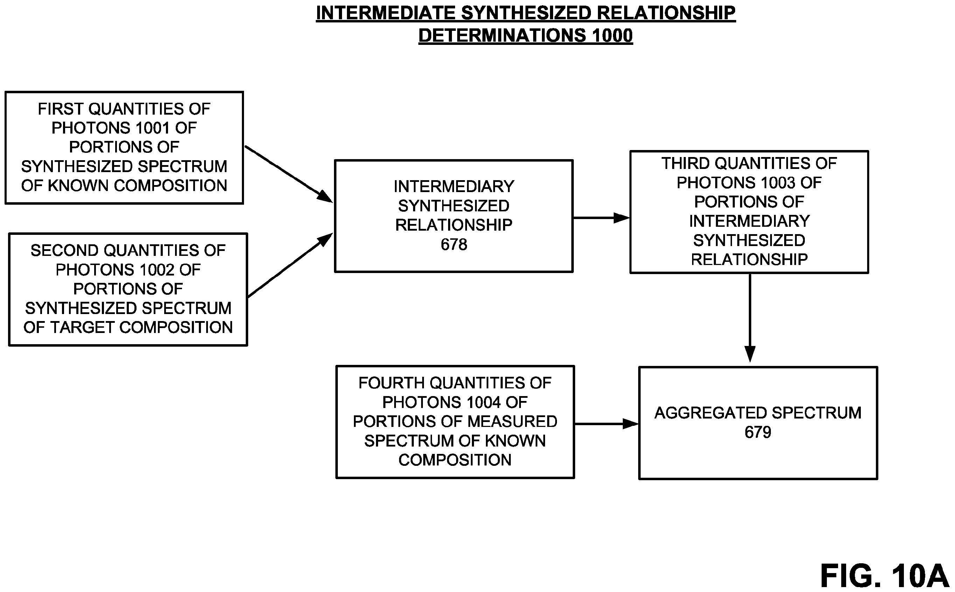

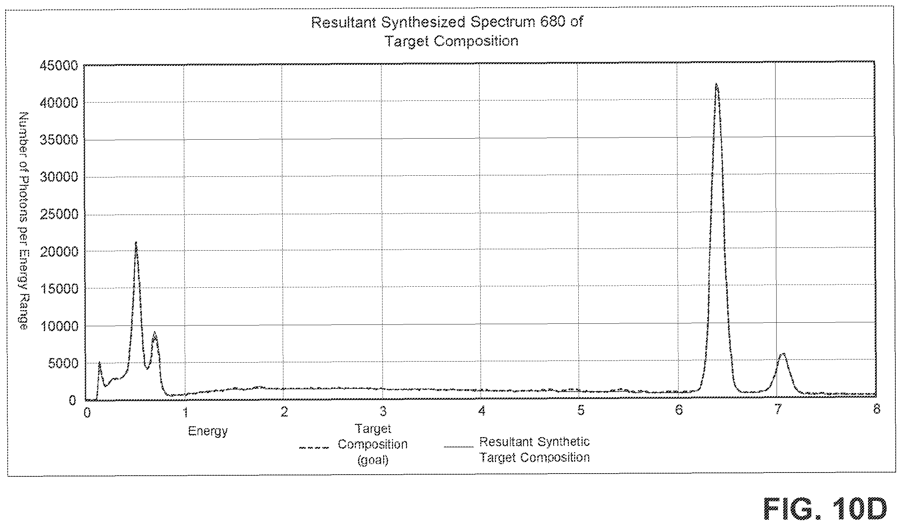

Discussion now turns to FIGS. 10A to 10D. Turning first to FIG. 10A to an illustrated process for the intermediate synthesized relationship determinations 1000, and still referring to FIG. 9, more particularly, the intermediary synthesized relationship 678 is based on a delta of first quantities of photons 1001 of portions of the baseline synthesized spectrum 658 of the known composition 652 and of second quantities of photons 1002 of portions of the initial synthesized spectrum 665 of the target composition 662. That is, the portions of the baseline synthesized spectrum 658 of the known composition 652 represent different energy ranges of the baseline synthesized spectrum 658 of the known composition 652, and the portions of the initial synthesized spectrum 665 of the target composition 662 represent different energy ranges of the initial synthesized spectrum 665 of the target composition 662.

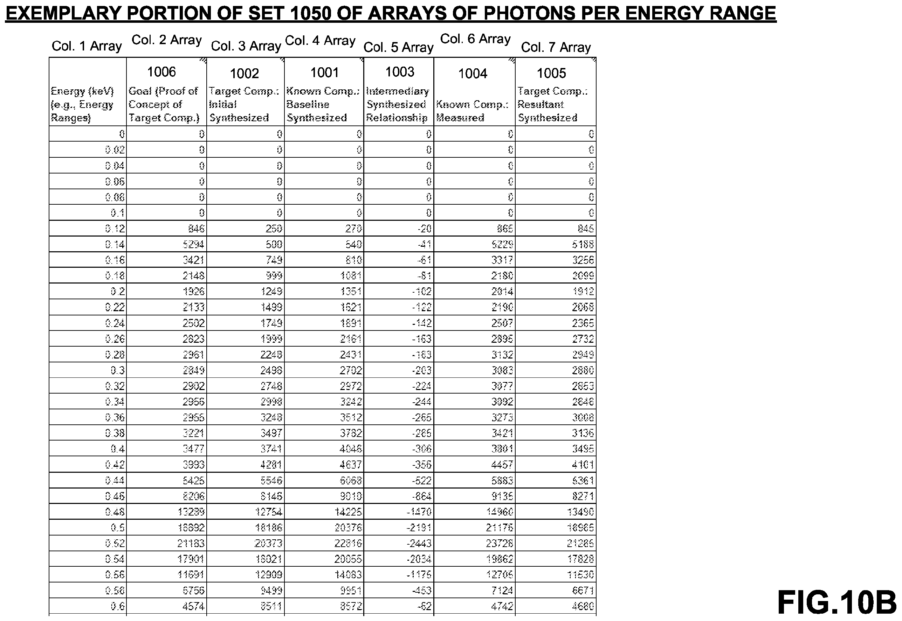

Even more particularly, determining the delta comprises determining a difference of a first array (e.g., column at FIG. 10B, or vector) of numbers, indexed over a set of energy ranges and corresponding to the first quantities of photons 1001, and a second array of numbers, indexed over the same set of energy ranges and corresponding to the second quantities of photons 1002.

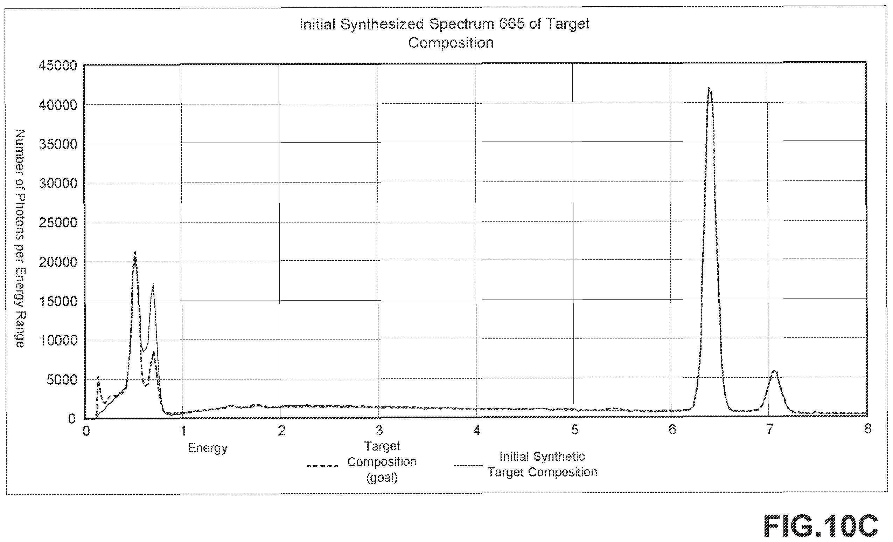

For example, turning to FIG. 10C, an exemplary initial synthesized spectrum 665 of a target composition 662 is illustrated. The x-axis denotes energy of the spectrum (e.g., in kiloelectron volts or keV), while the y-axis denotes numbers of photons at each energy of the x-axis. Data from this exemplary initial synthesized spectrum 665 of a target composition 662 can be transformed into an array of quantities of photons at each of a plurality of energy ranges of the energy of the x-axis. The energy ranges can be equal and the default range for each of the energy ranges can cover any suitable quantity. As illustrated at column 1 of the set 1050 of arrays of FIG. 10B, the energy ranges are set at 0.02 keV per energy range. Each of the arrays of quantities of photons 1001-1006 are indexed over the same set of energy ranges of column 1.

It is noted that the column 1 array extends only as far as 0.6 keV, rather than illustrating the full extent of energy ranges of the spectrum of FIG. 10C (the x-axis of which extends to 8.0 keV), for sake of brevity.

Also, while visualization of the set 1050 of arrays is useful to a user entity, and can be generated by the generation component 620, such as for display at the display region 302 (FIG. 3), generation of a visual version of the set 1050 of arrays is not required for determination of the intermediary synthesized relationship 678 or aggregated synthesized spectrum 680 by the generation component 620.

Next illustrated at FIG. 10B, at column 2, is an array of known quantities of photons 1006 of a measured spectrum of the target composition 662. While in use of the material analysis system 602, a measured spectrum or sample of established providence of the target composition may not be available (hence the use of the material analysis system 602), in the example of FIGS. 10A-10D, the array of column 2 provides a proof of concept as compared against a final result at column 7, which array of column 7 corresponds to the resultant synthetic spectrum 680 of the target composition 662.

Illustrated at column 3, of the set 1050 of arrays of FIG. 10B, is the array of the second quantities of photons 1002 of the portions (e.g., energy ranges) of the exemplary initial synthesized spectrum 665 of the target composition 662 obtained from the spectrum graph of FIG. 10C.

Also illustrated at FIG. 10B, at column 4, is the array of the first quantities of photons 1001 of the portions (e.g., energy ranges) of a corresponding baseline synthesized spectrum 658 of the known composition 652.

Column 5 at FIG. 10B is the array of the third quantities of photons 1003 of the portions (e.g., energy ranges) of the intermediary synthesized relationship 678. This column 5 array is the result (e.g., the delta) of subtraction of the first quantities of photons 1001 (of the synthesized spectrum 658 of the known composition 652) from the second quantities of photons 1002 (of the synthesized spectrum 665 of the target composition 662). That is, the delta or subtraction is performed by the generation component 620 is performed for each energy range (e.g., each row of the set 1050 of arrays) relative to columns 3 and 4. That is, each individual quantity of photons of 1001 (column 4) is subtracted from the respective/corresponding (e.g., same row) individual quantity of photons 1002 (column 3), which results in the quantities of photons 1003 of the column 5 array. Put another way, column 3 - column 4 = column 5 or Equation 6. the initial synthetized spectrum 665 of the target composition 662 - the baseline synthesized spectrum 658 of the known composition 652 = the intermediary synthetized relationshop 678.

Further, as will be detailed below, column 5 + column 6 = column 7 or Equation 7. the intermediary synthesized relationship 678 - the measured spectrum 656 of the known composition 652 = aggregated synthetized spectrum 679 = resultant synthesized spectrum 680 of the target composition 662.

More particularly, column 3 - column 4 + column 6 = column 7 or Equation 8. the initial synthesized spectrum 665 of the target composition 662 - the baseline synthesized spectrum 656 of the known composition 652 = aggregated synthesized spectrum 679 = resultant synthesized spectrum 680 of the target composition 662.

Turning next to the second step mentioned above, that aggregated synthesized spectrum 679 can be generated by the generation component 620 based on the intermediary synthesized relationship 678.

For generating the aggregated synthesized spectrum 679, the intermediary synthesized relationship 678 and the measured spectrum 656 of the known composition 652 can be employed. That is, the aggregated synthesized spectrum 679 is determined as being a sum of (e.g., aggregation of) these two aspects 678 and 656.

Referring still to both FIGS. 9 and 10A-10D, more particularly, the aggregated synthesized spectrum 679 is based on a sum of third quantities of photons 1003 of portions of the intermediary synthesized relationship 678 and fourth quantities of photons 1004 of portions of the measured spectrum 656 of the known composition 652. That is, the portions of the intermediary synthesized relationship 678 represent the set of energy ranges, and the portions of the measured spectrum 656 of the known composition 652 represent the different energy ranges of the known composition 652 (e.g., the same set of energy ranges).

Even more particularly, determining the sum comprises summing of a third array of numbers, indexed over a third energy range and corresponding to the third quantities of photons 1003 (column 5 of FIG. 10B) and a fourth array of numbers, indexed over a fourth energy range and corresponding to the fourth quantities of photons 1004 (column 6 of FIG. 10B).

As illustrated at FIG. 10B, column 6 is the array of the fourth quantities of photons 1004 of the portions (e.g., energy ranges) of a corresponding measured spectrum 656 of the known composition 652.

Then, column 7 is the array of fifth quantities of photons 1005 of the portions (e.g., energy ranges) of the aggregated spectrum 679 which is the resultant synthesized spectrum 680 of the target composition 662. Column 7 is the result of an aggregation (e.g., summation) performed by the generation component 620 of each individual quantity of photons 1003 of column 5 (e.g., at each row of the set 1050 of arrays) with each corresponding individual quantity of photons 1004 (column 6). As put above, column 5 + column 6 = column 7 or Equation 7.

It will be appreciated that in one or more embodiments, the generation component 620 need not separately calculate the intermediary synthesized relationship 678, but rather than omit such step relative to use of Equation 8.

Finally, from the quantities of photons 1005, the resultant synthesized spectrum 680 can be generated for outputting to a user entity of the materials analysis system 602.

Also, upon determination of the resultant synthesized spectrum 680 of the target composition 662, an optional fourth stage of identification can be performed. The fourth stage can comprise self-improvement of the material analysis system 602 by use of the resultant synthesized spectrum 680.

That is, turning next to the database maintenance component 622, this component can update data in a database of known compositions (e.g., the database 640). For example, the database 640 can include the measured spectrum 656 and synthesized spectrum 658 of the known composition 652, and the resultant synthesized spectrum 680 of the target composition 662 can be added to the database 640 by the database maintenance component 662. In this way, a user entity of the material analysis system 602 can build a private library of known compositions for use in future unknown composition (e.g., target composition) identification. For example, upon future iterations of use of the material analysis system 602, the determination component 614 can determine the resultant synthesized spectrum 680 of the target composition 662 as a second known composition for determining a resultant synthesized spectrum of a second target composition.

As a brief summary of the one or more embodiments described above, discussion now turns to illustrations 1100 and 1150 of FIG. 11. As illustrated, the material depicted at the existing approach result 1100 is identified as a single component 1102. This is in error relative to the actual physical sample. Rather, using the systems and methods described herein (e.g., the material analysis system 602), the described approach result 1150 more accurately illustrates that the unknown material comprises a plurality of components (e.g., at least two components 1152 and 1154) and does not include 100% weight percentage of only a single component. Indeed, this type of accurate identification can be important in mining and/or other material procurement fields.

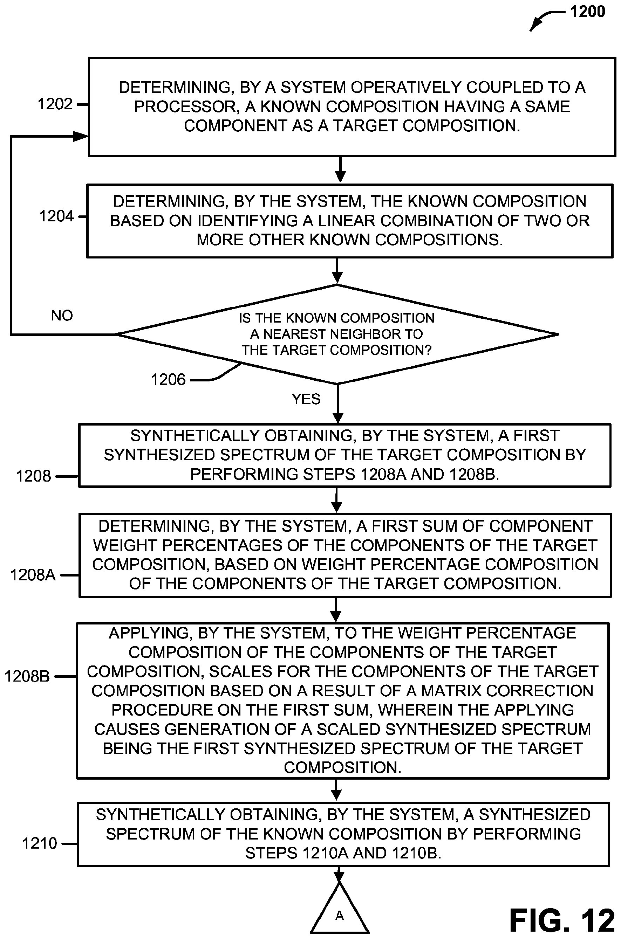

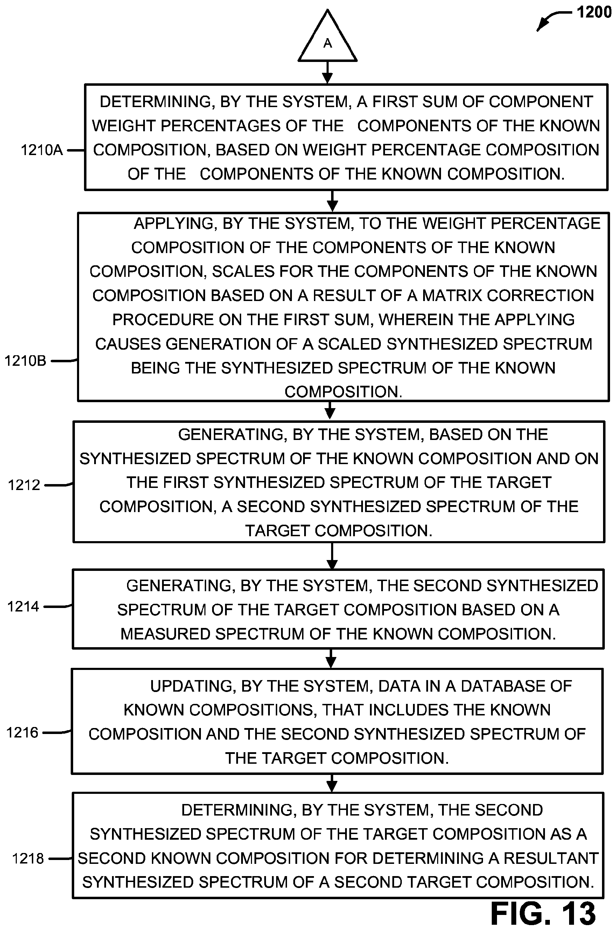

As another summary, referring next to FIGS. 12 and 13, illustrated is a flow diagram of an example, non-limiting method 1200 that can provide a process to generate a synthesized spectrum accurately defining a target composition, which synthesized spectrum is based on a nearest neighbor known composition, in accordance with one or more embodiments described herein, such as the non-limiting system 600 of FIG. 6. While the non-limiting method 1200 is described relative to the non-limiting system 600 of FIG. 6, the non-limiting method 1200 can be applicable also to other systems described herein, such as the non-limiting system 500 of FIG. 5. Repetitive description of like elements and/or processes employed in respective embodiments is omitted for sake of brevity.

At 1202, the non-limiting method 1200 can comprise determining, by a system operatively coupled to a processor (e.g., determination component 214), a known composition (e.g., known composition 652) having a same component (e.g., components 654) as a target composition (e.g., target composition 662 having components 664).

At 1204, the non-limiting method 1200 can comprise determining, by the system (e.g., linear combination component 616), the known composition based on identifying a linear combination of two or more other known compositions.

At 1206, the non-limiting method 1200 can comprise determining, by the system (e.g., determination component 614), whether the known composition is a nearest neighbor (e.g., nearest neighbor 710) of a set of known compositions (e.g., set of known compositions 650), including the known composition, to the target composition. In one or more embodiments, the nearest neighbor is a composition having component weight percentages closer to component weight percentages of the target composition than the other known compositions of the set of known compositions. If yes, the method can proceed to step 1208. If no, the method can proceed back to step 1202.

At 1208, the non-limiting method 1200 can comprise synthetically generating, by the system (e.g., synthesizing component 618), a first synthesized spectrum of the target composition (e.g., initial synthesized spectrum 665) by performing steps 1208A and 1208B.

At 1208A, the non-limiting method 1200 can comprise determining, by the system (e.g., synthesizing component 618), a first sum of component weight percentages of the components of the target composition, based on weight percentage composition of the components of the target composition.

1208B, the non-limiting method 1200 can comprise applying, by the system (e.g., synthesizing component 618), to the weight percentage composition of the components of the target composition, scales for the components of the target composition based on a result of a matrix correction procedure on the first sum, wherein the applying causes generation of a scaled synthesized spectrum being the first synthesized spectrum of the target composition.

At 1210, the non-limiting method 1200 can comprise synthetically generating, by the system (e.g., synthesizing component 618), a synthesized spectrum of the known composition (e.g., baseline synthesized spectrum 658) by performing steps 1210A and 1210B.

At 1210A, the non-limiting method 1200 can comprise determining, by the system (e.g., synthesizing component 618), a first sum of component weight percentages of the components of the known composition, based on weight percentage composition of the components of the known composition.

1210B, the non-limiting method 1200 can comprise applying, by the system (e.g., synthesizing component 618), to the weight percentage composition of the components of the known composition, scales for the components of the known composition based on a result of a matrix correction procedure on the first sum, wherein the applying causes generation of a scaled synthesized spectrum being the synthesized spectrum of the known composition.

At 1212, the non-limiting method 1200 can comprise generating, by the system (e.g., generation component 620), based on the synthesized spectrum of the known composition and on the first synthesized spectrum of the target composition, a second synthesized spectrum of the target composition (e.g., resultant synthesized spectrum 680 of the target composition 662).

At 1214, the non-limiting method 1200 can comprise generating, by the system (e.g., generation component 620), the second synthesized spectrum of the target composition based on a measured spectrum of the known composition (e.g., measured spectrum 656).

At 1216, the non-limiting method 1200 can comprise updating, by the system (e.g., database maintenance component 622), data in a database of known compositions (e.g., database 640), that includes the known composition and the second synthesized spectrum of the target composition.

At 1218, the non-limiting method 1200 can comprise determining, by the system (e.g., determination component 614), the second synthesized spectrum of the target composition as a second known composition for determining a resultant synthesized spectrum of a second target composition.

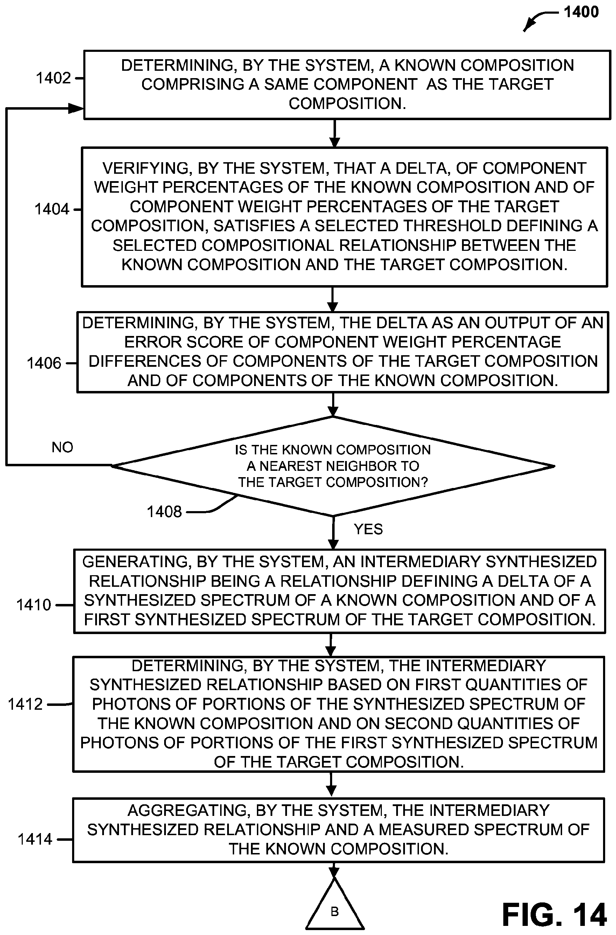

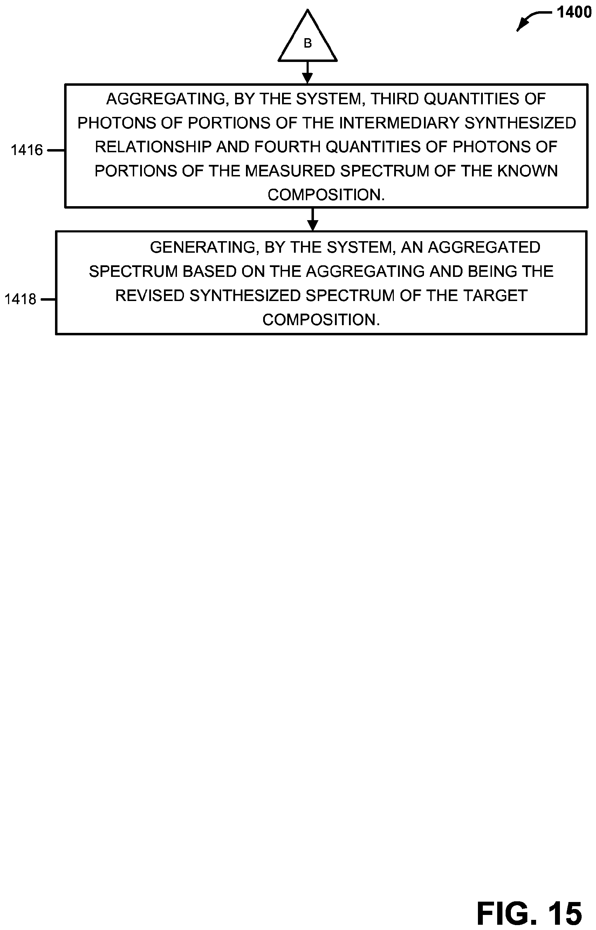

As another summary, referring next to FIGS. 14 and 15, illustrated is a flow diagram of an example, non-limiting method 1400 that can provide a process to synthesize, by a system operatively coupled to a processor, a revised synthesized spectrum (e.g., resultant synthesized spectrum 680) of a target composition (e.g., target composition 662), in accordance with one or more embodiments described herein, such as the non-limiting system 600 of FIG. 6. While the non-limiting method 1400 is described relative to the non-limiting system 600 of FIG. 6, the non-limiting method 1400 can be applicable also to other systems described herein, such as the non-limiting system 500 of FIG. 5. Repetitive description of like elements and/or processes employed in respective embodiments is omitted for sake of brevity.

At 1402, the non-limiting method 1400 can comprise determining, by the system (e.g., determination component 614), a known composition (e.g., known composition 652) comprising a same component (e.g., components 654 of the known composition and components 664 of the target composition) as the target composition. The known composition can comprise an atomic element, phase of an atomic element, or combination thereof.

At 1404, the non-limiting method 1400 can comprise verifying, by the system (e.g., determination component 614), that a delta, of component weight percentages of the known composition and of component weight percentages of the target composition, satisfies a selected threshold defining a selected compositional relationship between the known composition and the target composition.

At 1406, the non-limiting method 1400 can comprise determining, by the system (e.g., determination component 614), the delta as an output of an error score of component weight percentage differences of components of the target composition and of components of the known composition.

At 1408, the non-limiting method 1400 can comprise determining, by the system (e.g., determination component 614), whether the known composition is a nearest neighbor (e.g., nearest neighbor 710) of a set of known compositions (e.g., set of known compositions 650), including the known composition, to the target composition. In one or more embodiments, the nearest neighbor is a composition having component weight percentages closer to component weight percentages of the target composition than the other known compositions of the set of known compositions. If yes, the method can proceed to step 1410. If no, the method can proceed back to step 1402.

At 1410, the non-limiting method 1400 can comprise generating, by the system (e.g., generation component 620), an intermediary synthesized relationship (e.g., intermediary synthesized relationship 678) being a relationship defining a delta of a synthesized spectrum of a known composition (e.g., baseline synthesized spectrum 658) and of a first synthesized spectrum of the target composition (e.g., initial synthesized spectrum 665).

At 1412, the non-limiting method 1400 can comprise determining, by the system (e.g., generation component 620), the intermediary synthesized relationship based on first quantities of photons of portions of the synthesized spectrum of the known composition (e.g., first quantities of photons 1001) and on second quantities of photons of portions of the first synthesized spectrum of the target composition (e.g., second quantities of photons 1002).

At 1414, the non-limiting method 1400 can comprise aggregating, by the system (e.g., generation component 620), the intermediary synthesized relationship and a measured spectrum of the known composition (e.g., measured spectrum 656).

At 1416, the non-limiting method 1400 can comprise aggregating, by the system (e.g., generation component 620), third quantities of photons of portions of the intermediary synthesized relationship (e.g., third quantities of photons 1003) and fourth quantities of photons of portions of the measured spectrum of the known composition (e.g., fourth quantities of photons 1004).

At 1418, the non-limiting method 1400 can comprise generating, by the system (e.g., evaluation component 222), an aggregated synthesized spectrum (e.g., aggregated synthesized spectrum 679) based on the aggregating and being the revised synthesized spectrum of the target composition (e.g., resultant synthesized spectrum 680).

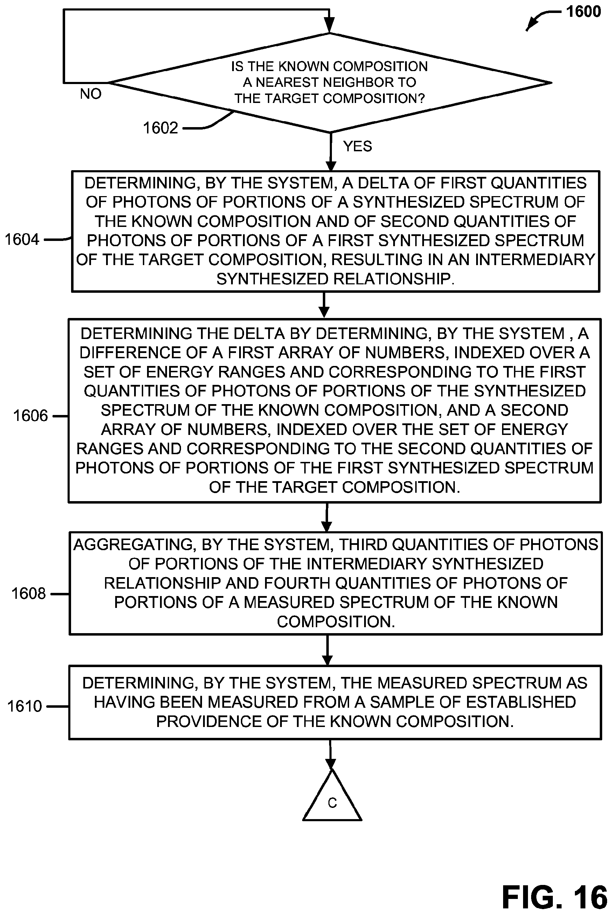

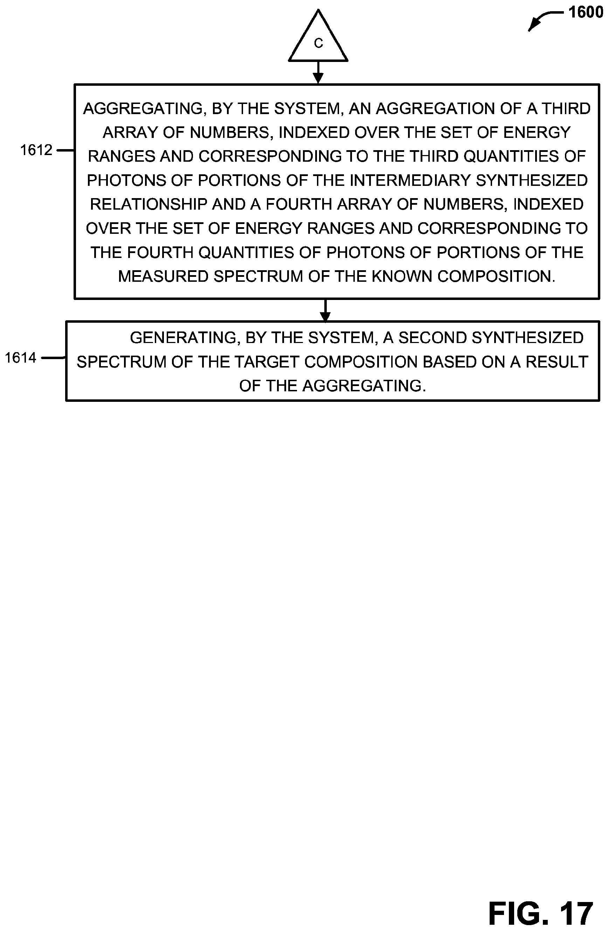

As another summary, referring next to FIGS. 16 and 17, illustrated is a flow diagram of an example, non-limiting method 1600 that can provide a process synthesize, by a system operatively coupled to a processor, a spectrum of a target composition (e.g., target composition 662), in accordance with one or more embodiments described herein, such as the non-limiting system 600 of FIG. 6. While the non-limiting method 1600 is described relative to the non-limiting system 600 of FIG. 6, the non-limiting method 1600 can be applicable also to other systems described herein, such as the non-limiting system 500 of FIG. 5. Repetitive description of like elements and/or processes employed in respective embodiments is omitted for sake of brevity.

At 1602, the non-limiting method 1600 can comprise determining, by the system (e.g., determination component 614), whether a known composition (e.g., known composition 652) is a nearest neighbor (e.g., nearest neighbor 710) of a set of known compositions (e.g., set of known compositions 650), including the known composition, to the target composition. In one or more embodiments, the nearest neighbor is a composition having component weight percentages closer to component weight percentages of the target composition than the other known compositions of the set of known compositions. If yes, the method can proceed to step 1604. If no, the method can repeat step 1602.

At 1604, the non-limiting method 1600 can comprise determining, by the system (e.g., generation component 620), a delta of first quantities of photons of portions of a synthesized spectrum of the known composition (e.g., first quantities of photons 1001 of baseline synthesized spectrum 558 of known composition 652) and of second quantities of photons of portions of a first synthesized spectrum of the target composition (e.g., second quantities of photons 1002 of initial synthesized spectrum 665 of target composition 662), resulting in an intermediary synthesized relationship (e.g., intermediary synthesized relationship 678). In one or more embodiments, the portions of the synthesized spectrum of the known composition represent different energy ranges of the synthesized spectrum of the known composition and the portions of the synthesized spectrum of the target composition represent different energy ranges of the synthesized spectrum of the target composition.

At 1606, the non-limiting method 1600 can comprise determining the delta by determining, by the system (e.g., generation component 620), a difference of a first array of numbers, indexed over a set of energy ranges and corresponding to the first quantities of photons of portions of the synthesized spectrum of the known composition, and a second array of numbers, indexed over the set of energy ranges and corresponding to the second quantities of photons of portions of the first synthesized spectrum of the target composition.

At 1608, the non-limiting method 1600 can comprise aggregating, by the system (e.g., generation component 620), third quantities of photons of portions of the intermediary synthesized relationship (e.g., third quantities of photons 1003) and fourth quantities of photons of portions of a measured spectrum of the known composition (e.g., fourth quantities of photons 1004 of the measured spectrum 656 of the known composition 652). In one or more embodiments, the portions of the intermediary synthesized relationship represent different energy ranges of the intermediary synthesized relationship, and the portions of the measured spectrum of the known composition represent different energy ranges of the measured spectrum of the known composition.

At 1610, the non-limiting method 1600 can comprise determining, by the system (e.g., determination component 614), the measured spectrum as having been measured from a sample of established providence of the known composition.

At 1612, the non-limiting method 1600 can comprise aggregating, by the system (e.g., generation component 620), an aggregation of a third array of numbers, indexed over the set of energy ranges and corresponding to the third quantities of photons of portions of the intermediary synthesized relationship and a fourth array of numbers, indexed over the set of energy ranges and corresponding to the fourth quantities of photons of portions of the measured spectrum of the known composition.

At 1614, the non-limiting method 1600 can comprise generating, by the system (e.g., generation component 620), a second synthesized spectrum (e.g., resultant synthesized spectrum 680) of the target composition based on a result of the aggregating.

### Additional Summary

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. In addition, the computer-implemented and non-computer-implemented methodologies could alternatively be represented as a series of interrelated states via a state diagram or events. Additionally, the computer-implemented methodologies described hereinafter and throughout this specification are capable of being stored on an article of manufacture for transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In summary, one or more systems, computer program products and/or computer-implemented methods provided herein relate to synthetically generating a synthesized spectrum for a target composition. A system can comprise a memory that stores computer executable components and a processor that executes the computer executable components stored in the memory. The computer executable components can comprise a determination component that determines a known composition having a same component as a target composition, and a generation component that, based on a synthesized spectrum of the known composition and on a first synthesized spectrum of the target composition, generates a second synthesized spectrum of the target composition.

Indeed, in view of the one or more embodiments described herein, a practical application of the one or more systems, computer-implemented methods and/or computer program products described herein can be ability to quickly and efficiently generate a synthesized spectrum of a target composition, even where a measured spectrum of such target composition would comprise two or more significant peaks. The one or more embodiments described herein can improve upon existing approaches to achieve the technical advantages of high accuracy identification of material compositions by using known compositions, and particularly the measured and synthetically-generated spectra thereof, as baselines for generating a highly accurate spectrum defining a target composition. Then, by using this synthetically-generated spectrum (i.e., not a measured spectrum), accurate identification of the target composition can be made. This can be achieved without generating a measured spectrum of the target composition from a sample of established providence, since providence of the target composition itself has not yet been established.

Furthermore, in one or more embodiments, synthetically-generated spectra can be employed for a next known composition for subsequent identifications of subsequent target compositions. That is, a library of synthetically-derived spectra can be built and employed for further material analysis use, thus allowing for the system to self-improve by becoming more accurate for target composition analysis and identification.

These are useful and practical applications of computers, thus providing enhanced (e.g., improved and/or optimized) data analysis and prediction output. Overall, such computerized tools can constitute a concrete and tangible technical improvement in the fields of material analysis, and more particularly in material analysis using EDS.

Furthermore, one or more embodiments described herein can be employed in a real-world system based on the disclosed teachings. For example, as noted above, even in cases where a measured spectrum of the target composition has multiple significant peaks, the one or more embodiments described herein can accurately identify the target composition and enable the synthetic generation of the spectrum for the target composition. The embodiments disclosed herein thus provide improvements to scientific instrument technology (e.g., improvements in the computer technology supporting such scientific instruments, among other improvements).

Moreover, a device and/or method described herein can be implemented in one or more domains to enable scaled synthesized spectrum output. Indeed, use of a system as described herein can be scalable, such as where plural inputs target compositions can be evaluated and plural resultant synthesized spectra for the target compositions can be generated at least partially at a same time as one another.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

One or more embodiments described herein can be, in one or more embodiments, inherently and/or inextricably tied to computer technology and cannot be implemented outside of a computing environment. For example, one or more processes performed by one or more embodiments described herein can more efficiently, and even more feasibly, provide program and/or program instruction execution, such as relative to material analysis based on nearest neighbor known compositions and spectra thereof, as compared to existing systems and/or techniques. Systems, computer-implemented methods and/or computer program products providing performance of these processes are of great utility in the fields of material analysis, such as comprising EDS and cannot be equally practicably implemented in a sensible way outside of a computing environment.

One or more embodiments described herein can employ hardware and/or software to solve problems that are highly technical, that are not abstract, and that cannot be performed as a set of mental acts by a human. For example, a human, or even thousands of humans, cannot efficiently, accurately and/or effectively automatically determine a nearest neighbor known composition, generate initial synthesized spectra and/or generate a resultant synthesized spectrum absent a material sample of known providence as the one or more embodiments described herein can provide this process. Moreover, neither can the human mind nor a human with pen and paper conduct one or more of these processes, as conducted by one or more embodiments described herein.

In one or more embodiments, one or more of the processes described herein can be performed by one or more specialized computers (e.g., a specialized processing unit, a specialized classical computer, a specialized quantum computer, a specialized hybrid classical/quantum system and/or another type of specialized computer) to execute defined tasks related to the one or more technologies describe above. One or more embodiments described herein and/or components thereof can be employed to solve new problems that arise through advancements in technologies mentioned above, employment of quantum computing systems, cloud computing systems, computer architecture and/or another technology.

One or more embodiments described herein can be fully operational towards performing one or more other functions (e.g., fully powered on, fully executed and/or another function) while also performing one or more of the one or more operations described herein.

To provide additional summary, a listing of embodiments and features thereof is next provided.

A system comprises a memory that stores computer executable components; and a processor that executes the computer executable components stored in the memory, wherein the computer executable components comprise: a determination component that determines a known composition having a same component as a target composition; and a generation component that, based on a synthesized spectrum of the known composition and on a first synthesized spectrum of the target composition, generates a second synthesized spectrum of the target composition.

The system of the preceding paragraph, wherein the generation component further generates the second synthesized spectrum of the target composition based on a measured spectrum of the known composition.

The system of any preceding paragraph, wherein the determination component determines the known composition as being a nearest neighbor of a set of known compositions, including the known composition, to the target composition, and wherein the nearest neighbor is a composition having component weight percentages closer to component weight percentages of the target composition than the other known compositions of the set of known compositions.

The system of any preceding paragraph, further comprising: a linear combination component that further determines the known composition based on identifying a linear combination of two or more other known compositions.

The system of any preceding paragraph, further comprising: a synthesizing component that synthetically generates the first synthesized spectrum of the target composition by performing steps comprising: determining a first sum of component weight percentages of the components of the target composition, based on weight percentage composition of the components of the target composition; and applying, to the weight percentage composition of the components of the target composition, scales for the components of the target composition based on a result of a matrix correction procedure on the first sum, wherein the applying causes generation of a scaled synthesized spectrum being the first synthesized spectrum of the target composition.

The system of any preceding paragraph, further comprising: a synthesizing component that synthetically generates the synthesized spectrum of the known composition by performing steps comprising: determining a first sum of component weight percentages of the components of the known composition, based on weight percentage composition of the components of the known composition; and applying, to the weight percentage composition of the components of the known composition, scales for the components of the known composition based on a result of a matrix correction procedure on the first sum, wherein the applying causes generation of a scaled synthesized spectrum being the synthesized spectrum of the known composition.

The system of any preceding paragraph, further comprising: a database maintenance component that updates data in a database of known compositions, that includes the measured spectrum and the synthesized spectrum of the known composition and the second synthesized spectrum of the target composition, wherein the determination component determines the second synthesized spectrum of the target composition as a second known composition for determining a resultant synthesized spectrum of a second target composition.

A computer-implemented method, comprises synthesizing, by a system operatively coupled to a processor, a revised synthesized spectrum of a target composition, comprising: generating, by the system, an intermediary synthesized relationship being a relationship defining a delta of a synthesized spectrum of a known composition and of a first synthesized spectrum of the target composition; aggregating, by the system, the intermediary synthesized relationship and a measured spectrum of the known composition; and generating, by the system, an aggregated synthesized spectrum based on the aggregating and being the revised synthesized spectrum of the target composition.

The computer-implemented method of the preceding paragraph, wherein the known composition comprises a same component as the target composition, and wherein the same component comprises an atomic element, molecular element, phase of an atomic or molecular element, or combination thereof.

The computer-implemented method of any preceding paragraph, further comprising: determining, by the system, the known composition as being a nearest neighbor of a set of known compositions, including the known composition, to the target composition, and wherein the nearest neighbor is a composition having component weight percentages closer to component weight percentages of the target composition than the other known compositions of the set of known compositions.

The computer-implemented method of any preceding paragraph, wherein the generating the intermediary synthesized relationship comprises determining the intermediary synthesized relationship based on first quantities of photons of portions of the synthesized spectrum of the known composition and on second quantities of photons of portions of the first synthesized spectrum of the target composition.

The computer-implemented method of any preceding paragraph, wherein the aggregating further comprises aggregating third quantities of photons of portions of the intermediary synthesized relationship and fourth quantities of photons of portions of the measured spectrum of the known composition.

The computer-implemented method of any preceding paragraph, further comprising: verifying, by the system, that a delta, of component weight percentages of the known composition and of component weight percentages of the target composition, satisfies a selected threshold defining a selected compositional relationship between the known composition and the target composition.

The computer-implemented method of any preceding paragraph, wherein the synthesizing the spectrum of the target composition further comprises: determining, by the system, the delta as an output of an error score of component weight percentage differences of components of the target composition and of components of the known composition.

A computer program product facilitates a process for synthesizing a spectrum of a target composition, wherein the program instructions are executable by a processor to cause the processor to: synthesize, by the processor, a spectrum of a target composition, wherein the synthesizing comprises causing the processor to: determine, by the processor, a delta of first quantities of photons of portions of a synthesized spectrum of a known composition and of second quantities of photons of portions of a first synthesized spectrum of the target composition, resulting in an intermediary synthesized relationship; aggregate, by the processor, third quantities of photons of portions of the intermediary synthesized relationship and fourth quantities of photons of portions of a measured spectrum of the known composition; and generate, by the processor, a second synthesized spectrum of the target composition based on a result of the aggregating.

The computer program product of the preceding paragraph, wherein the portions of the synthesized spectrum of the known composition represent different energy ranges of the synthesized spectrum of the known composition, wherein the portions of the synthesized spectrum of the target composition represent different energy ranges of the synthesized spectrum of the target composition, wherein the portions of the intermediary synthesized relationship represent different energy ranges of the intermediary synthesized relationship, and wherein the portions of the measured spectrum of the known composition represent different energy ranges of the measured spectrum of the known composition.

The computer program product of any preceding paragraph, wherein the determining the delta comprises determining a difference of a first array of numbers, indexed over a set of energy ranges and corresponding to the first quantities of photons of portions of the synthesized spectrum of the known composition, and a second array of numbers, indexed over the set of energy ranges and corresponding to the second quantities of photons of portions of the first synthesized spectrum of the target composition.

The computer program product of any preceding paragraph, wherein the aggregating comprises determining an aggregation of a third array of numbers, indexed over the set of energy ranges and corresponding to the third quantities of photons of portions of the intermediary synthesized relationship and a fourth array of numbers, indexed over the set of energy ranges and corresponding to the fourth quantities of photons of portions of the measured spectrum of the known composition.

The computer program product of any preceding paragraph, wherein the measured spectrum has been measured from a sample of established providence of the known composition.

The computer program product of any preceding paragraph, wherein the program instructions are further executable by the processor to cause the processor to:

determine, by the processor, the known composition as being a nearest neighbor of a set of known compositions, including the known composition, to the target composition, and wherein the nearest neighbor is a composition having component weight percentages closer to component weight percentages of the target composition than the other known compositions of the set of known compositions.

### Scientific Instrument System Description

Turning next to FIG. 18, a detailed description is provided of additional context for the one or more embodiments described herein at FIGS. 1-17. One or more computing devices implementing any of the scientific instrument modules or methods disclosed herein can be part of a scientific instrument system. FIG. 18 illustrates a block diagram of an example scientific instrument system 1800 in which one or more of the scientific instrument methods or other methods disclosed herein can be performed, in accordance with various embodiments described herein. The scientific instrument modules and methods disclosed herein (e.g., the scientific instrument module 100 of FIG. 1 and the method 200 of FIG. 2) can be implemented by one or more of the scientific instrument 1810, the user local computing device 1820, the service local computing device 1830, and/or the remote computing device 1840 of the scientific instrument system 1800.

Any of the scientific instrument 1810, the user local computing device 1820, the service local computing device 1830, and/or the remote computing device 1840 can include any of the embodiments of the computing device 400 discussed herein with reference to FIG. 4, and any of the scientific instrument 1810, the user local computing device 1820, the service local computing device 1830, and/or the remote computing device 1840 can take the form of any appropriate one or more of the embodiments of the computing device 400 discussed herein with reference to FIG. 4.

One or more of the scientific instrument 1810, the user local computing device 1820, the service local computing device 1830, and/or the remote computing device 1840 can include a processing device 1802, a storage device 1804, and/or an interface device 1806. The processing device 1802 can take any suitable form, including the form of any of the processors 402 discussed herein with reference to FIG. 4. The processing devices 1802 included in different ones of the scientific instrument 1810, the user local computing device 1820, the service local computing device 1830, and/or the remote computing device 1840 can take the same form or different forms. The storage device 1804 can take any suitable form, including the form of any of the storage devices 404 discussed herein with reference to FIG. 4. The storage devices 1804 included in different ones of the scientific instrument 1810, the user local computing device 1820, the service local computing device 1830, and/or the remote computing device 1840 can take the same form or different forms. The interface device 1806 can take any suitable form, including the form of any of the interface devices 406 discussed herein with reference to FIG. 4. The interface devices 1806 included in different ones of the scientific instrument 1810, the user local computing device 1820, the service local computing device 1830, and/or the remote computing device 1840 can take the same form or different forms.

The scientific instrument 1810, the user local computing device 1820, the service local computing device 1830, and/or the remote computing device 1840 can be in communication with other elements of the scientific instrument system 1800 via communication pathways 1808. The communication pathways 1808 can communicatively couple the interface devices 1806 of different ones of the elements of the scientific instrument system 1800, as shown, and can be wired or wireless communication pathways (e.g., in accordance with any of the communication techniques discussed herein with reference to the interface devices 406 of the computing device 400 of FIG. 4). The particular scientific instrument system 1800 depicted in FIG. 18 includes communication pathways between each pair of the scientific instrument 1810, the user local computing device 1820, the service local computing device 1830, and the remote computing device 1840, but this "fully connected" implementation is simply illustrative, and in various embodiments, various ones of the communication pathways 1808 can be omitted. For example, in one or more embodiments, a service local computing device 1830 can omit a direct communication pathway 1808 between its interface device 1806 and the interface device 1806 of the scientific instrument 1810, but can instead communicate with the scientific instrument 1810 via the communication pathway 1808 between the service local computing device 1830 and the user local computing device 1820 and/or the communication pathway 1808 between the user local computing device 1820 and the scientific instrument 1810.

The scientific instrument 1810 can include any appropriate scientific instrument, such as an EDS device or device associated with or connected to an EDS device.

The user local computing device 1820 can be a computing device (e.g., in accordance with any of the embodiments of the computing device 400 discussed herein) that is local to a user of the scientific instrument 1810. In one or more embodiments, the user local computing device 1820 can also be local to the scientific instrument 1810, but this need not be the case; for example, a user local computing device 1820 that is associated with a home, office or other building associated with a user entity can be remote from, but in communication with, the scientific instrument 1810 so that the user entity can use the user local computing device 1820 to control and/or access data from the scientific instrument 1810. In one or more embodiments, the user local computing device 1820 can be a laptop, smartphone, or tablet device. In one or more embodiments the user local computing device 1820 can be a portable computing device. In one or more embodiments, the user local computing device 1820 can deployed in the field.

The service local computing device 1830 can be a computing device (e.g., in accordance with any of the embodiments of the computing device 400 discussed herein) that is local to an entity that services the scientific instrument 1810. For example, the service local computing device 1830 can be local to a manufacturer of the scientific instrument 1810 or to a third-party service company. In one or more embodiments, the service local computing device 1830 can communicate with the scientific instrument 1810, the user local computing device 1820, and/or the remote computing device 1840 (e.g., via a direct communication pathway 1808 or via multiple "indirect" communication pathways 1808, as discussed above) to receive data regarding the operation of the scientific instrument 1810, the user local computing device 1820, and/or the remote computing device 1840 (e.g., the results of self-tests of the scientific instrument 1810, calibration coefficients used by the scientific instrument 1810, the measurements of sensors associated with the scientific instrument 1810, etc.). In one or more embodiments, the service local computing device 1830 can communicate with the scientific instrument 1810, the user local computing device 1820, and/or the remote computing device 1840 (e.g., via a direct communication pathway 1808 or via multiple "indirect" communication pathways 1808, as discussed above) to transmit data to the scientific instrument 1810, the user local computing device 1820, and/or the remote computing device 1840 (e.g., to update programmed instructions, such as firmware, in the scientific instrument 1810, to initiate the performance of test or calibration sequences in the scientific instrument 1810, to update programmed instructions, such as software, in the user local computing device 1820 or the remote computing device 1840, etc.). A user entity of the scientific instrument 1810 can utilize the scientific instrument 1810 or the user local computing device 1820 to communicate with the service local computing device 1830 to report a problem with the scientific instrument 1810 or the user local computing device 1820, to request a visit from a technician to improve the operation of the scientific instrument 1810, to order consumables or replacement parts associated with the scientific instrument 1810, or for other purposes.

The remote computing device 1840 can be a computing device (e.g., in accordance with any of the embodiments of the computing device 400 discussed herein) that is remote from the scientific instrument 1810 and/or from the user local computing device 1820. In one or more embodiments, the remote computing device 1840 can be included in a datacenter or other large-scale server environment. In one or more embodiments, the remote computing device 1840 can include network-attached storage (e.g., as part of the storage device 1804). The remote computing device 1840 can store data generated by the scientific instrument 1810, perform analyses of the data generated by the scientific instrument 1810 (e.g., in accordance with programmed instructions), facilitate communication between the user local computing device 1820 and the scientific instrument 1810, and/or facilitate communication between the service local computing device 1830 and the scientific instrument 1810.

In one or more embodiments, one or more of the elements of the scientific instrument system 1800 illustrated in FIG. 18 can be omitted. Further, in one or more embodiments, multiple ones of various ones of the elements of the scientific instrument system 1800 of FIG. 18 can be present. For example, a scientific instrument system 1800 can include multiple user local computing devices 1820 (e.g., different user local computing devices 1820 associated with different user entities or in different locations). In another example, a scientific instrument system 1800 can include multiple scientific instruments 1810, all in communication with service local computing device 1830 and/or a remote computing device 1840; in such an embodiment, the service local computing device 1830 can monitor these multiple scientific instruments 1810, and the service local computing device 1830 can cause updates or other information can be "broadcast" to multiple scientific instruments 1810 at the same time. Different ones of the scientific instruments 1810 in a scientific instrument system 1800 can be located close to one another (e.g., in the same room) or farther from one another (e.g., on different floors of a building, in different buildings, in different cities, etc.). In one or more embodiments, a scientific instrument 1810 can be connected to an Internet-of-Things (IoT) stack that allows for command and control of the scientific instrument 1810 through a web-based application, a virtual or augmented reality application, a mobile application, and/or a desktop application. Any of these applications can be accessed by a user entity operating the user local computing device 1820 in communication with the scientific instrument 1810 by the intervening remote computing device 1840. In one or more embodiments, a scientific instrument 1810 can be sold by the manufacturer along with one or more associated user local computing devices 1820 as part of a local scientific instrument computing unit 1812.

In one or more embodiments, different ones of the scientific instruments 1810 included in a scientific instrument system 1800 can be different types of scientific instruments 1810; for example, one scientific instrument 1810 can be an EDS device, while another scientific instrument 1810 can be an analysis device that analyzes results of an EDS device. In some such embodiments, the remote computing device 1840 and/or the user local computing device 1820 can combine data from different types of scientific instruments 1810 included in a scientific instrument system 1800.

### Example Operating Environment

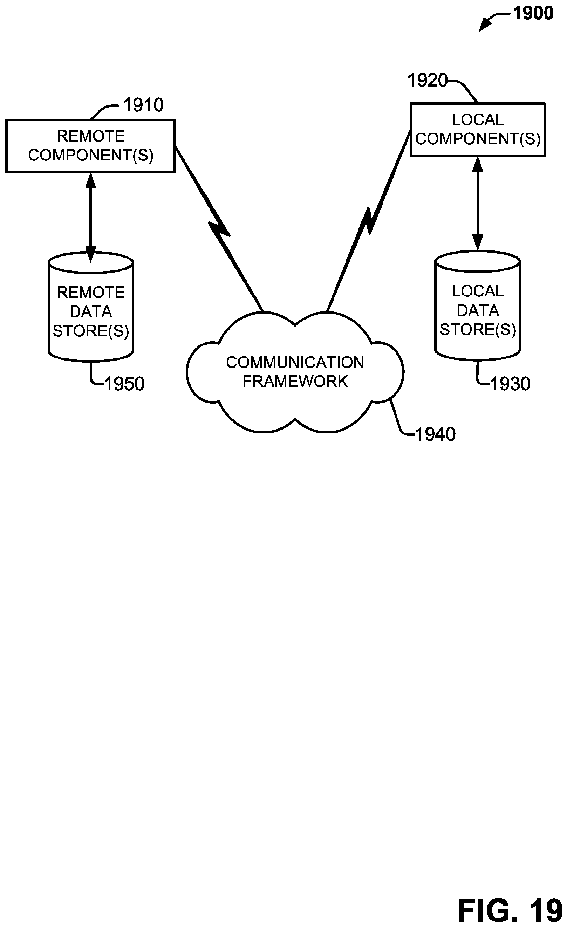

FIG. 19 is a schematic block diagram of an operating environment 1900 with which the described subject matter can interact. The operating environment 1900 comprises one or more remote component(s) 1910. The remote component(s) 1910 can be hardware and/or software (e.g., threads, processes, computing devices). In one or more embodiments, remote component(s) 1910 can be a distributed computer system, connected to a local automatic scaling component and/or programs that use the resources of a distributed computer system, via communication framework 1940. Communication framework 1940 can comprise wired network devices, wireless network devices, mobile devices, wearable devices, radio access network devices, gateway devices, femtocell devices, servers, etc.

The operating environment 1900 also comprises one or more local component(s) 1920. The local component(s) 1920 can be hardware and/or software (e.g., threads, processes, computing devices). In one or more embodiments, local component(s) 1920 can comprise an automatic scaling component and/or programs that communicate/use the remote resources 1910 and 1920, etc., connected to a remotely located distributed computing system via communication framework 1940.

One possible communication between a remote component(s) 1910 and a local component(s) 1920 can be in the form of a data packet adapted to be transmitted between two or more computer processes. Another possible communication between a remote component(s) 1910 and a local component(s) 1920 can be in the form of circuit-switched data adapted to be transmitted between two or more computer processes in radio time slots. The operating environment 1900 comprises a communication framework 1940 that can be employed to facilitate communications between the remote component(s) 1910 and the local component(s) 1920, and can comprise an air interface, e.g., interface of a UMTS network, via an LTE network, etc. Remote component(s) 1910 can be operably connected to one or more remote data store(s) 1950, such as a hard drive, solid state drive, subscriber identity module (SIM) card, electronic SIM (eSIM), device memory, etc., that can be employed to store information on the remote component(s) 1910 side of communication framework 1940. Similarly, local component(s) 1920 can be operably connected to one or more local data store(s) 1930, that can be employed to store information on the local component(s) 1920 side of communication framework 1940.

### Example Computing Environment

In order to provide additional context for various embodiments described herein, FIG. 20 and the following discussion are intended to provide a brief, general description of a suitable computing environment 2000 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform tasks or implement abstract data types. Moreover, the methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

Referring still to FIG. 20, the example computing environment 2000 which can implement one or more embodiments described herein includes a computer 2002, the computer 2002 including a processing unit 2004, a system memory 2006 and a system bus 2008. The system bus 2008 couples system components including, but not limited to, the system memory 2006 to the processing unit 2004. The processing unit 2004 can be any of various commercially available processors. Dual microprocessors and other multi processor architectures can also be employed as the processing unit 2004.

The system bus 2008 can be any of several types of bus structure that can further interconnect to a memory bus (with or without a memory controller), a peripheral bus, and a local bus using any of a variety of commercially available bus architectures. The system memory 2006 includes ROM 2010 and RAM 2012. A basic input/output system (BIOS) can be stored in a non-volatile memory such as ROM, erasable programmable read only memory (EPROM), EEPROM, which BIOS contains the basic routines that help to transfer information between elements within the computer 2002, such as during startup. The RAM 2012 can also include a high-speed RAM such as static RAM for caching data.

The computer 2002 further includes an internal hard disk drive (HDD) 2014 (e.g., EIDE, SATA), and can include one or more external storage devices 2016 (e.g., a magnetic floppy disk drive (FDD) 2016, a memory stick or flash drive reader, a memory card reader, etc.). While the internal HDD 2014 is illustrated as located within the computer 2002, the internal HDD 2014 can also be configured for external use in a suitable chassis (not shown). Additionally, while not shown in computing environment 2000, a solid-state drive (SSD) could be used in addition to, or in place of, an HDD 2014.

Other internal or external storage can include at least one other storage device 2020 with storage media 2022 (e.g., a solid-state storage device, a nonvolatile memory device, and/or an optical disk drive that can read or write from removable media such as a CD-ROM disc, a DVD, a BD, etc.). The external storage 2016 can be facilitated by a network virtual machine. The HDD 2014, external storage device 2016 and storage device (e.g., drive) 2020 can be connected to the system bus 2008 by an HDD interface 2024, an external storage interface 2026 and a drive interface 2028, respectively.

The drives and their associated computer-readable storage media provide nonvolatile storage of data, data structures, computer-executable instructions, and so forth. For the computer 2002, the drives and storage media accommodate the storage of any data in a suitable digital format. Although the description of computer-readable storage media above refers to respective types of storage devices, other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 2012, including an operating system 2030, one or more application programs 2032, other program modules 2034 and program data 2036. All or portions of the operating system, applications, modules, and/or data can also be cached in the RAM 2012. The systems and methods described herein can be implemented utilizing various commercially available operating systems or combinations of operating systems.

Computer 2002 can optionally comprise emulation technologies. For example, a hypervisor (not shown) or other intermediary can emulate a hardware environment for operating system 2030, and the emulated hardware can optionally be different from the hardware illustrated in FIG. 20. In such an embodiment, operating system 2030 can comprise one virtual machine (VM) of multiple VMs hosted at computer 2002. Furthermore, operating system 2030 can provide runtime environments, such as the Java runtime environment or the .NET framework, for applications 2032. Runtime environments are consistent execution environments that allow applications 2032 to run on any operating system that includes the runtime environment. Similarly, operating system 2030 can support containers, and applications 2032 can be in the form of containers, which are lightweight, standalone, executable packages of software that include, e.g., code, runtime, system tools, system libraries and settings for an application.

Further, computer 2002 can be enabled with a security module, such as a trusted processing module (TPM). For instance, with a TPM, boot components hash next in time boot components, and wait for a match of results to secured values, before loading a next boot component. This process can take place at any layer in the code execution stack of computer 2002, e.g., applied at the application execution level or at the operating system (OS) kernel level, thereby enabling security at any level of code execution.

A user entity can enter commands and information into the computer 2002 through one or more wired/wireless input devices, e.g., a keyboard 2038, a touch screen 2040, and a pointing device, such as a mouse 2042. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera, a gesture sensor input device, a vision movement sensor input device, an emotion or facial detection device, a biometric input device, e.g., fingerprint or iris scanner, or the like. These and other input devices are often connected to the processing unit 2004 through an input device interface 2044 that can be coupled to the system bus 2008, but can be connected by other interfaces, such as a parallel port, an IEEE 1394 serial port, a game port, a USB port, an IR interface, a BLUETOOTHO interface, etc.

A monitor 2046 or other type of display device can also be connected to the system bus 2008 via an interface, such as a video adapter 2048. In addition to the monitor 2046, a computer typically includes other peripheral output devices (not shown), such as speakers, printers, etc.

The computer 2002 can operate in a networked environment using logical connections via wired and/or wireless communications to one or more remote computers, such as a remote computer 2050. The remote computer 2050 can be a workstation, a server computer, a router, a personal computer, portable computer, microprocessor-based entertainment appliance, a peer device or other common network node, and typically includes many or all of the elements described relative to the computer 2002, although, for purposes of brevity, only a memory/storage device 2052 is illustrated. The logical connections depicted include wired/wireless connectivity to a local area network (LAN) 2054 and/or larger networks, e.g., a wide area network (WAN) 2056. Such LAN and WAN networking environments are commonplace in offices and companies, and facilitate enterprise-wide computer networks, such as intranets, all of which can connect to a global communications network, e.g., the Internet.

When used in a LAN networking environment, the computer 2002 can be connected to the local network 2054 through a wired and/or wireless communication network interface or adapter 2058. The adapter 2058 can facilitate wired or wireless communication to the LAN 2054, which can also include a wireless access point (AP) disposed thereon for communicating with the adapter 2058 in a wireless mode.

When used in a WAN networking environment, the computer 2002 can include a modem 2060 or can be connected to a communications server on the WAN 2056 via other means for establishing communications over the WAN 2056, such as by way of the Internet. The modem 2060, which can be internal or external and a wired or wireless device, can be connected to the system bus 2008 via the input device interface 2044. In a networked environment, program modules depicted relative to the computer 2002 or portions thereof, can be stored in the remote memory/storage device 2052. The network connections shown are example and other means of establishing a communications link between the computers can be used.

When used in either a LAN or WAN networking environment, the computer 2002 can access cloud storage systems or other network-based storage systems in addition to, or in place of, external storage devices 2016 as described above. Generally, a connection between the computer 2002 and a cloud storage system can be established over a LAN 2054 or WAN 2056 e.g., by the adapter 2058 or modem 2060, respectively. Upon connecting the computer 2002 to an associated cloud storage system, the external storage interface 2026 can, with the aid of the adapter 2058 and/or modem 2060, manage storage provided by the cloud storage system as it would other types of external storage. For instance, the external storage interface 2026 can be configured to provide access to cloud storage sources as if those sources were physically connected to the computer 2002.

The computer 2002 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a defined structure as with an existing network or simply an ad hoc communication between at least two devices.

### Additional Closing Information

The embodiments described herein can be directed to one or more of a system, a method, an apparatus and/or a computer program product at any possible technical detail level of integration. The computer program product can include a computer readable storage medium (or media) having computer readable program instructions thereon for causing a processor to carry out aspects of the one or more embodiments described herein. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a superconducting storage device and/or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, is not to be construed as being transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein can be downloaded to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more embodiments, electronic circuitry including, for example, programmable logic circuitry, field-programmable gate arrays (FPGA) and/or programmable logic arrays (PLA) can execute the computer readable program instructions by utilizing state information of the computer readable program instructions to personalize the electronic circuitry, in order to perform aspects of the one or more embodiments described herein.

Aspects of the one or more embodiments described herein are described with reference to flowchart illustrations and/or block diagrams of methods, apparatus (systems), and computer program products according to one or more embodiments described herein. It will be understood that each block of the flowchart illustrations and/or block diagrams, and combinations of blocks in the flowchart illustrations and/or block diagrams, can be implemented by computer readable program instructions. These computer readable program instructions can be provided to a processor of a general-purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computerimplementable methods and/or computer program products according to one or more embodiments described herein. In this regard, each block in the flowchart or block diagrams can represent a module, segment and/or portion of instructions, which comprises one or more executable instructions for implementing the specified logical function. In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter has been described above in the general context of computer-executable instructions of a computer program product that runs on a computer and/or computers, those skilled in the art will recognize that the one or more embodiments herein also can be implemented at least partially in parallel with one or more other program modules. Generally, program modules include routines, programs, components and/or data structures that perform particular tasks and/or implement particular abstract data types. Moreover, the aforedescribed computer-implemented methods can be practiced with other computer system configurations, including single-processor and/or multiprocessor computer systems, mini-computing devices, mainframe computers, as well as computers, hand-held computing devices (e.g., PDA, phone), and/or microprocessor-based or programmable consumer and/or industrial electronics. The illustrated aspects can also be practiced in distributed computing environments in which tasks are performed by remote processing devices that are linked through a communications network. However, one or more, if not all aspects of the one or more embodiments described herein can be practiced on standalone computers. In a distributed computing environment, program modules can be located in both local and remote memory storage devices.

As used in this application, the terms "component," "system," "platform" and/or "interface" can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form. As used herein, the terms "example" and/or "exemplary" are utilized to mean serving as an example, instance, or illustration. For the avoidance of doubt, the subject matter described herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

As it is employed in the subject specification, the term "processor" can refer to substantially any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nano-scale architectures such as, but not limited to, molecular and quantum-dot based transistors, switches and/or gates, in order to optimize space usage and/or to enhance performance of related equipment. A processor can be implemented as a combination of computing processing units.

Herein, terms such as "store," "storage," "data store," data storage," "database," and substantially any other information storage component relevant to operation and functionality of a component are utilized to refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory and/or nonvolatile randomaccess memory (RAM) (e.g., ferroelectric RAM (FeRAM). Volatile memory can include RAM, which can act as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein are intended to include, without being limited to including, these and/or any other suitable types of memory.

What has been described above includes mere examples of systems and computer-implemented methods. It is, of course, not possible to describe every conceivable combination of components and/or computer-implemented methods for purposes of describing the one or more embodiments, but one of ordinary skill in the art can recognize that many further combinations and/or permutations of the one or more embodiments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the various embodiments can use the phrases "an embodiment," "various embodiments," "one or more embodiments" and/or "some embodiments," each of which can refer to one or more of the same or different embodiments.

The descriptions of the various embodiments have been presented for purposes of illustration but are not intended to be exhaustive or limited to the embodiments described herein. Many modifications and variations will be apparent to those of ordinary skill in the art without departing from the scope and spirit of the described embodiments. The terminology used herein was chosen to best explain the principles of the embodiments, the practical application and/or technical improvement over technologies found in the marketplace, and/or to enable others of ordinary skill in the art to understand the embodiments described herein.

## Claims

1. A system, comprising:
a memory that stores computer executable components; and
a processor that executes the computer executable components stored in the memory, wherein the computer executable components comprise:
a determination component that determines a known composition having a same component as a target composition; and
a generation component that, based on a synthesized spectrum of the known composition and on a first synthesized spectrum of the target composition, generates a second synthesized spectrum of the target composition.

2. The system of claim 1, wherein the generation component further generates the second synthesized spectrum of the target composition based on a measured spectrum of the known composition.

3. The system of any of the preceding claims,
wherein the determination component determines the known composition as being a nearest neighbor of a set of known compositions, including the known composition, to the target composition, and
wherein the nearest neighbor is a composition having component weight percentages closer to component weight percentages of the target composition than the other known compositions of the set of known compositions.

4. The system of any of the preceding claims, further comprising:
a linear combination component that further determines the known composition based on identifying a linear combination of two or more other known compositions.

5. The system of any of the preceding claims, further comprising:
a synthesizing component that synthetically generates the first synthesized spectrum of the target composition by performing steps comprising:
determining a first sum of component weight percentages of the components of the target composition, based on weight percentage composition of the components of the target composition; and
applying, to the weight percentage composition of the components of the target composition, scales for the components of the target composition based on a result of a matrix correction procedure on the first sum,
wherein the applying causes generation of a scaled synthesized spectrum being the first synthesized spectrum of the target composition.

6. The system of any of the preceding claims, further comprising:
a synthesizing component that synthetically generates the synthesized spectrum of the known composition by performing steps comprising:
determining a first sum of component weight percentages of the components of the known composition, based on weight percentage composition of the components of the known composition; and
applying, to the weight percentage composition of the components of the known composition, scales for the components of the known composition based on a result of a matrix correction procedure on the first sum,
wherein the applying causes generation of a scaled synthesized spectrum being the synthesized spectrum of the known composition.

7. The system of any of the preceding claims, further comprising:
a database maintenance component that updates data in a database of known compositions, that includes the measured spectrum and the synthesized spectrum of the known composition and the second synthesized spectrum of the target composition,
wherein the determination component determines the second synthesized spectrum of the target composition as a second known composition for determining a resultant synthesized spectrum of a second target composition.

8. A computer-implemented method, comprising:
synthesizing, by a system operatively coupled to a processor, a revised synthesized spectrum of a target composition, comprising:
generating, by the system, an intermediary synthesized relationship being a relationship defining a delta of a synthesized spectrum of a synthesized spectrum of a known composition and a first synthesized spectrum of the target composition;
aggregating, by the system, the intermediary synthesized relationship and a measured spectrum of the known composition; and
generating, by the system, an aggregated synthesized spectrum based on the aggregating and being the revised synthesized spectrum of the target composition.

9. The computer-implemented method of claim 8,
wherein the known composition comprises a same component as the target composition, and wherein the same component comprises an atomic element, molecular element, phase of an atomic or molecular element, or combination thereof.

10. The computer-implemented method of any of claims 8 and 9,
wherein the generating the intermediary synthesized relationship comprises determining the intermediary synthesized relationship based on first quantities of photons of portions of the synthesized spectrum of the known composition and on second quantities of photons of portions of the first synthesized spectrum of the target composition.

11. The computer-implemented method of any of claims 8 - 10,
wherein the aggregating further comprises aggregating third quantities of photons of portions of the intermediary synthesized relationship and fourth quantities of photons of portions of the measured spectrum of the known composition.

12. The computer-implemented method of any of claims 8- 11, further comprising:
verifying, by the system, that a delta, of component weight percentages of the known composition and of component weight percentages of the target composition, satisfies a selected threshold defining a selected compositional relationship between the known composition and the target composition.

13. A computer program product facilitating a process for synthesizing a spectrum of a target composition, the program instructions executable by a processor to cause the processor to:
synthesize, by the processor, a spectrum of a target composition, wherein the synthesizing comprises causing the processor to:
determine, by the processor, a delta of first quantities of photons of portions of a synthesized spectrum of a known composition and of second quantities of photons of portions of a first synthesized spectrum of the target composition, resulting in an intermediary synthesized relationship;
aggregate, by the processor, third quantities of photons of portions of the intermediary synthesized relationship and fourth quantities of photons of portions of a measured spectrum of the known composition; and
generate, by the processor, a second synthesized spectrum of the target composition based on a result of the aggregating.

14. The computer program product of claim 13,
wherein the portions of the synthesized spectrum of the known composition represent different photon energy ranges of the synthesized spectrum of the known composition,
wherein the portions of the synthesized spectrum of the target composition represent different photon energy ranges of the synthesized spectrum of the target composition,
wherein the portions of the intermediary synthesized relationship represent different photon energy ranges of the intermediary synthesized relationship, and
wherein the portions of the measured spectrum of the known composition represent different photon energy ranges of the measured spectrum of the known composition.

15. The computer program product of any of claims 13 and 14,
wherein the determining the delta comprises determining a difference of a first array of numbers, indexed over a set of energy ranges and corresponding to the first quantities of photons of portions of the synthesized spectrum of the known composition, and a second array of numbers, indexed over the set of energy ranges and corresponding to the second quantities of photons of portions of the first synthesized spectrum of the target composition, and/or
wherein the aggregating comprises determining an aggregation of a third array of numbers, indexed over the set of energy ranges and corresponding to the third quantities of photons of portions of the intermediary synthesized relationship and a fourth array of numbers, indexed over the set of energy ranges and corresponding to the fourth quantities of photons of portions of the measured spectrum of the known composition.
